# EUROPEAN PATENT APPLICATION

(11) **EP 4 349 957 A1**
(43) Date of publication of application: **10.04.2024**
(21) Application number: 22811599.4
(22) Date of filing: 24.05.2022
(51) Int. Cl.: C12M 3/00, C12M 1/12, C12M 3/06, C12M 1/00, C12N 5/071

(54) **NON-ALCOHOLIC FATTY LIVER ARTIFICIAL TISSUE MODEL**

(30) Priority: 24.05.2021 KR 20210066220; 23.05.2022 KR 20220062798
(71) Applicant: Cellartgen Inc., Seoul 03722 (KR)
(72) Inventor: CHO, Seung Woo, Seoul 03722 (KR); KIM, Su Kyeom, Seoul 04208 (KR); CUI, Baofang, Seoul 03726 (KR); KIM, Jin, Seoul 03722 (KR); BAE, Soo Han, Seoul 03722 (KR); HAN, Dai Hoon, Seoul 03722 (KR)
(74) Representative: Manitz Finsterwald Patent- und Rechtsanwaltspartnerschaft mbB
(86) International application number: PCT/KR2022/007325
(87) International publication number: WO 2022/250406

(57) **Abstract**

The present disclosure relates to a non-alcoholic fatty liver artificial tissue model. As compared to a conventional technology by which tissues are cultured only in Matrigel including a device composed of a decellularized liver tissue-derived extracellular matrix and a plurality of microchannels or a decellularized liver tissue-derived extracellular matrix, the present disclosure enables better mimicking of an actual non-alcoholic fatty liver disease due to the presence of Kupffer cells and hepatic stellate cells. Also, according to the present disclosure, the growth of liver organoids can be improved and fat accumulation and inflammation in the liver organoids can be caused to occur well through free fatty acid treatment, and the phenotypes of non-alcoholic fatty liver can be better expressed.

## Description

### TECHNICAL FIELD

The present disclosure relates to a non-alcoholic fatty liver artificial tissue model.

### BACKGROUND

Non-alcoholic fatty liver (NAFL) disease, whose basic lesion is fatty liver, is a disease state which exhibits tissue changes of the hepatic parenchyma such as inflammation, necrosis, and fibrosis that are similar to those in alcoholic liver injury, even without a long history of alcohol intake. NAFLD is basically asymptomatic. During the course of progression of the disease state, the fatty liver leads to steatohepatitis, liver cirrhosis, and then to liver cancer. The steatohepatitis in NAFLD is called Non-Alcoholic SteatoHepatitis (NASH). In particular, in recent years, metabolic syndromes due to obesity or diabetes have become a social problem, and NASH is also regarded as one of the metabolic syndromes. As complications of NAFLD and NASH, lifestyle-related diseases such as obesity, diabetes, hyperlipidemia, and hypertension are found, and their major clinical manifestations characteristically include increases in the blood alanine aminotransferase (ALT) and hyaluronic acid levels, and on the other hand, decreases in the total blood cholesterol and albumin levels. However, the pathogenic mechanisms of NAFLD and NASH still remain largely unclear, and there is no established effective therapeutic method or therapeutic drug therefor at present. This is partly due to the fact that, since development of NAFLD and NASH is based on lifestyle-related diseases of humans, non-human models suitable for research of NAFLD and NASH have not been established yet.

Elucidation of the disease states of NAFLD and NASH, which may progress to fatal diseases such as liver cirrhosis and liver cancer, is indispensable for development of effective therapeutic methods and therapeutic drugs therefor, and the elucidation requires appropriate models for NAFLD and NASH.

Animal models for NASH have so far been reported (for example, Patent Document 1). However, non-human animal models for NAFLD have been hardly reported. Further, due to recent problems related to animal ethics, the need for developing an in vitro model as an alternative to animal models has increased.

Thus, the inventors of the present disclosure completed the present disclosure as a result of researching the non-alcoholic fatty liver model.

### DISCLOSURE OF THE INVENTION

### PROBLEMS TO BE SOLVED BY THE INVENTION

An aspect of the present disclosure is conceived to provide a non-alcoholic fatty liver artificial tissue model, including: a hydrogel containing a decellularized liver tissue-derived extracellular matrix (Liver Extracellular Matrix; LEM); a liver organoid; a device including a well in which the hydrogel is located and a plurality of microchannels through which a free fatty acid flows; and a culture medium containing a free fatty acid.

Another aspect of the present disclosure is conceived to provide a method of fabricating a non-alcoholic fatty liver artificial tissue model, including: a process of fabricating the non-alcoholic fatty liver artificial tissue model; and a process of perfusing the non-alcoholic fatty liver artificial tissue model with a culture medium containing a free fatty acid.

Yet another aspect of the present disclosure is conceived to provide a method of screening a therapeutic drug for a non-alcoholic fatty liver disease, including: a process of treating the non-alcoholic fatty liver artificial tissue model with a candidate substance; and a process of comparing a group treated with the candidate substance and a control group.

### MEANS FOR SOLVING THE PROBLEMS

An aspect of the present disclosure provides a non-alcoholic fatty liver artificial tissue model, including: a hydrogel containing a decellularized liver tissue-derived extracellular matrix (Liver Extracellular Matrix; LEM); a liver organoid; a device including a well in which the hydrogel is located and a plurality of microchannels through which a free fatty acid flows; and a culture medium containing a free fatty acid.

In an embodiment of the present disclosure, the LEM may be a matrix in which 95% to 99.9% of liver tissue cells have been removed.

In an embodiment of the present disclosure, the liver organoid may be derived from a human induced pluripotent stem cell (hiPSC) or human liver tissue.

In an embodiment of the present disclosure, when the liver organoid is derived from the human induced pluripotent stem cell (hiPSC), the liver organoid may be cultured on the hydrogel.

In an embodiment of the present disclosure, when the liver organoid is derived from the human liver tissue, the liver organoid may be cultured within the hydrogel.

In an embodiment of the present disclosure, the well in which the hydrogel is located may have a depth of 2 mm to 4 mm.

In an embodiment of the present disclosure, the well in which the hydrogel is located may have a depth of 0.5 mm to 1.5 mm.

In an embodiment of the present disclosure, the free fatty acid may have a concentration of 100 µM to 900 µM.

In an embodiment of the present disclosure, the liver organoid may be co-cultured with any one or more of vascular cells, mesenchymal stem cells, Kupffer cells (KC), and hepatic stellate cells (HSC).

Another aspect of the present disclosure provides a method of fabricating a non-alcoholic fatty liver artificial tissue model, including: a process of fabricating the non-alcoholic fatty liver artificial tissue model; and a process of perfusing the non-alcoholic fatty liver artificial tissue model with a culture medium containing a free fatty acid.

In an embodiment of the present disclosure, the process of perfusing may be performed by shaking the non-alcoholic fatty liver artificial tissue model on a rocking shaker.

Yet another aspect of the present disclosure provides a method of screening a therapeutic drug for a non-alcoholic fatty liver disease, including: a process of treating the non-alcoholic fatty liver artificial tissue model with a candidate substance; and a process of comparing a group treated with the candidate substance and a control group.

### EFFECTS OF THE INVENTION

As compared to a conventional technology by which tissues are cultured only in Matrigel including a device composed of a decellularized liver tissue-derived extracellular matrix and a plurality of microchannels or a decellularized liver tissue-derived extracellular matrix, a non-alcoholic fatty liver artificial tissue model of the present disclosure enables better mimicking of an actual non-alcoholic fatty liver disease due to the presence of Kupffer cells and hepatic stellate cells. Also, with the non-alcoholic fatty liver artificial tissue model, the growth of liver organoids can be improved and fat accumulation and inflammation in the liver organoids can be caused to occur well through free fatty acid treatment, and the non-alcoholic fatty liver can be better mimicked.

Further, since the non-alcoholic fatty liver artificial tissue model of the present disclosure mimics the non-alcoholic fatty liver, it can be used for screening a therapeutic drug for a non-alcoholic fatty liver disease.

### BRIEF DESCRIPTION OF THE DRAWINGS

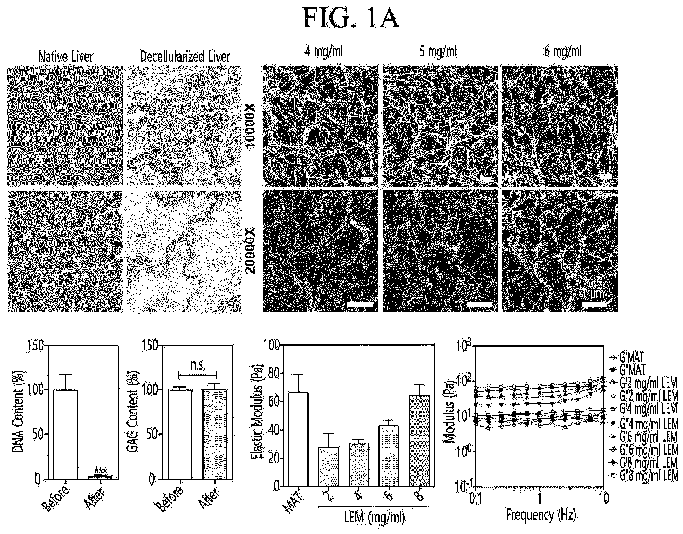

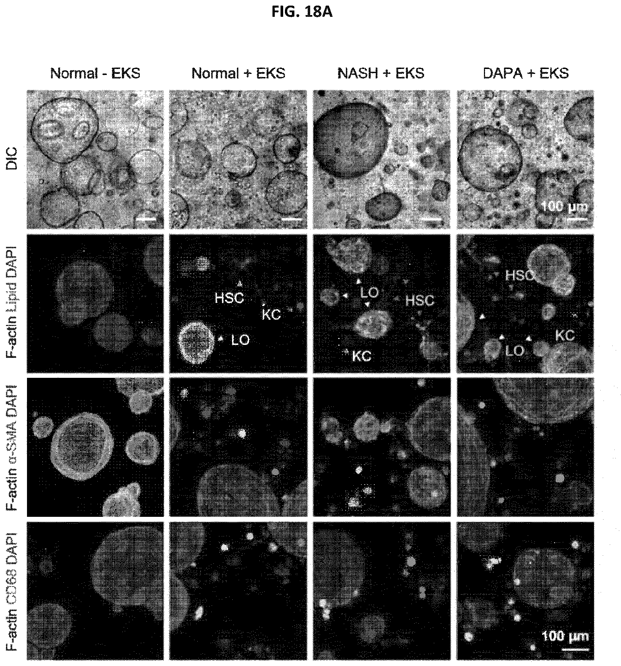

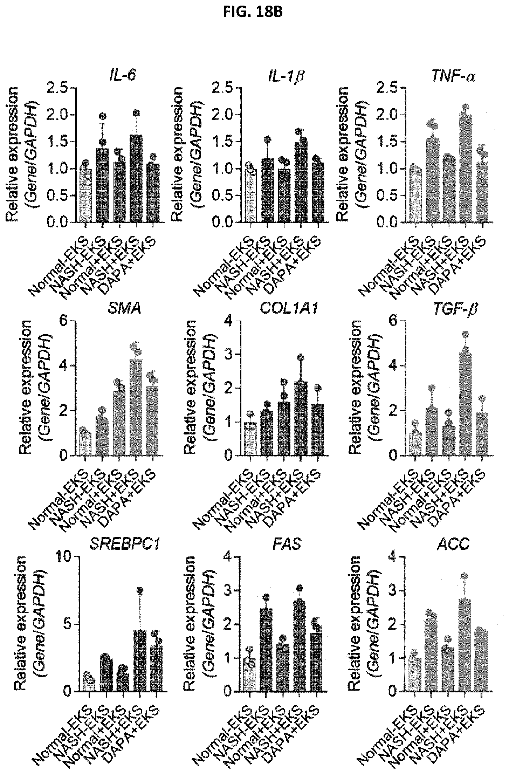

**FIG. 1A** and **FIG. 18** show the results of analyzing a decellularized liver tissue-derived extracellular matrix (LEM).

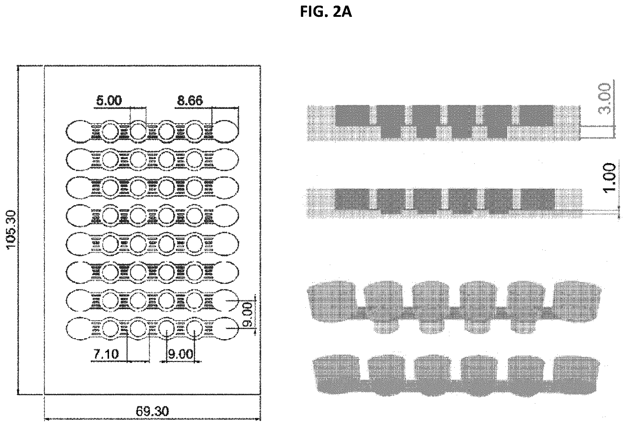

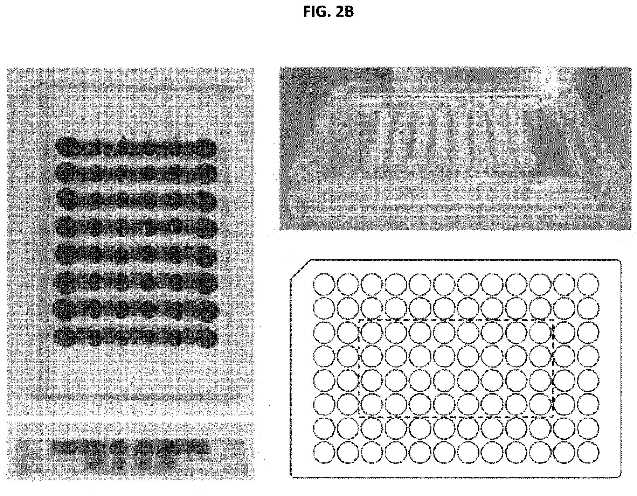

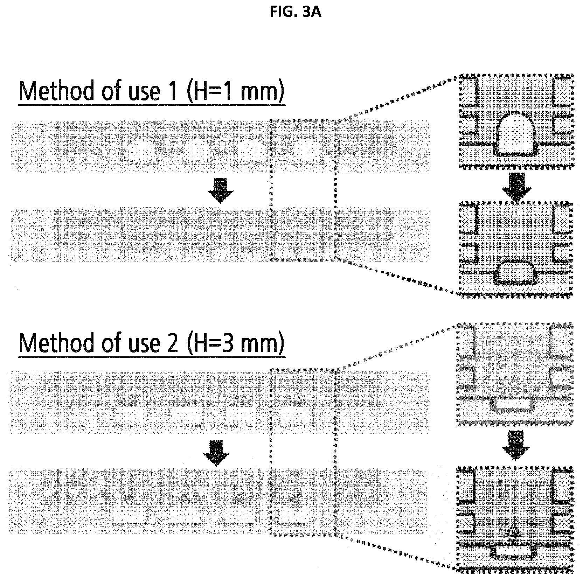

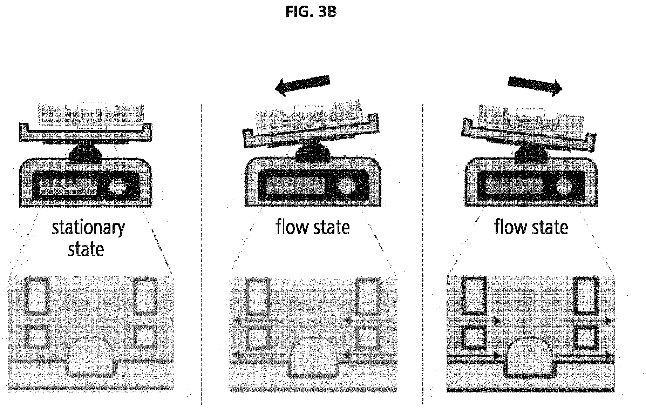

**FIG. 2A** and **FIG. 2B** are diagrams showing the configuration of a device for fabricating a non-alcoholic fatty liver artificial tissue model. **FIG. 3A** and **FIG. 3B** are diagrams showing the operation method of the device for fabricating a non-alcoholic fatty liver artificial tissue model.

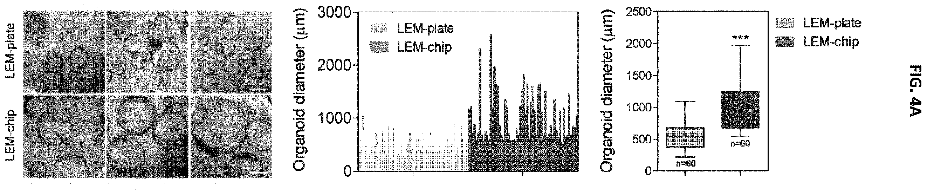

**FIG. 4A** to **FIG. 4C** show the results of culturing liver organoids in the device of the present disclosure.

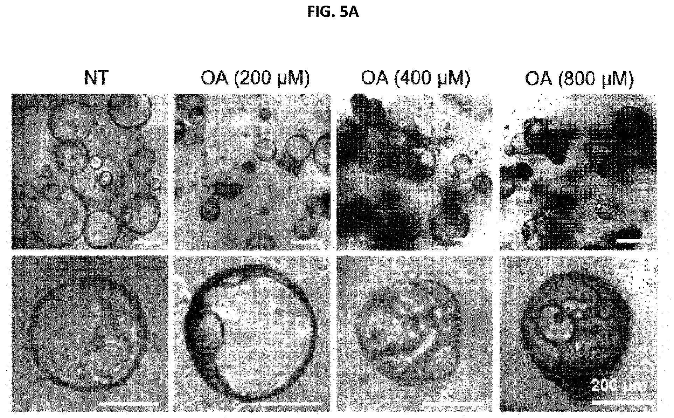

**FIG. 5A to FIG. 5C** show the results of tests for optimizing the treatment concentration of a free fatty acid.

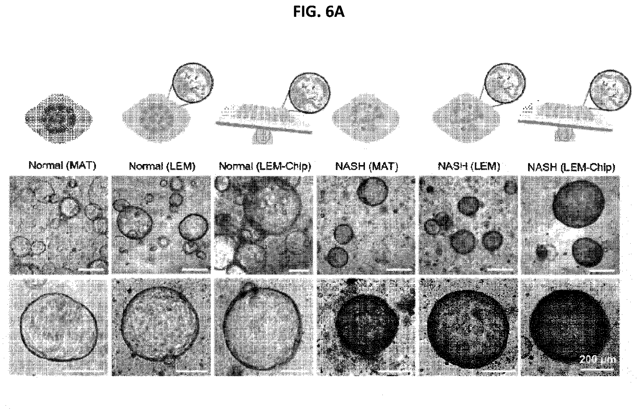

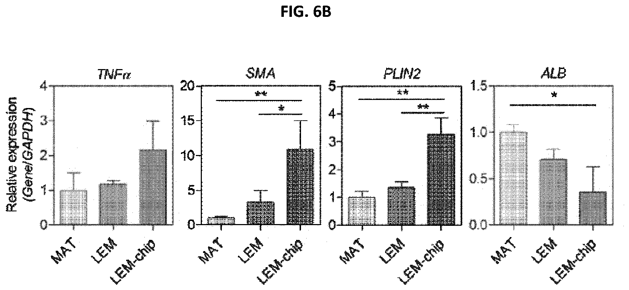

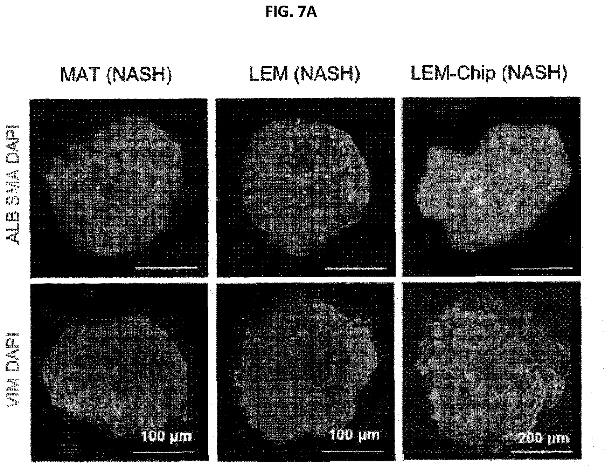

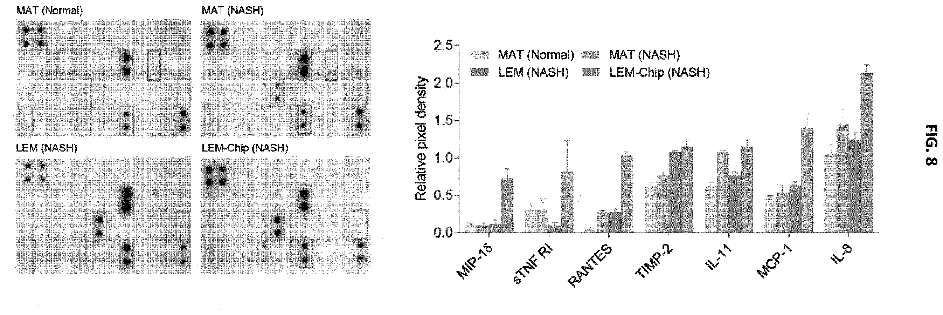

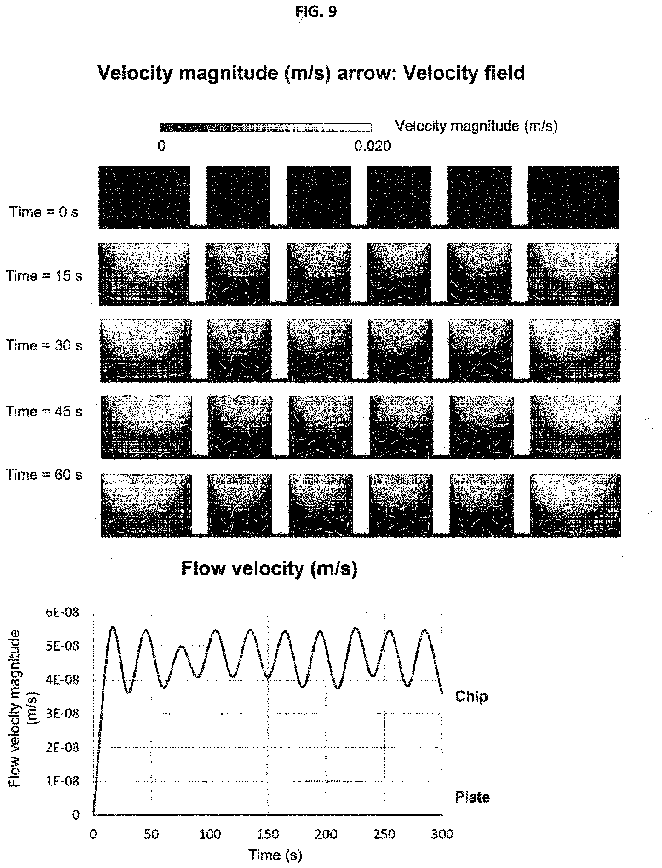

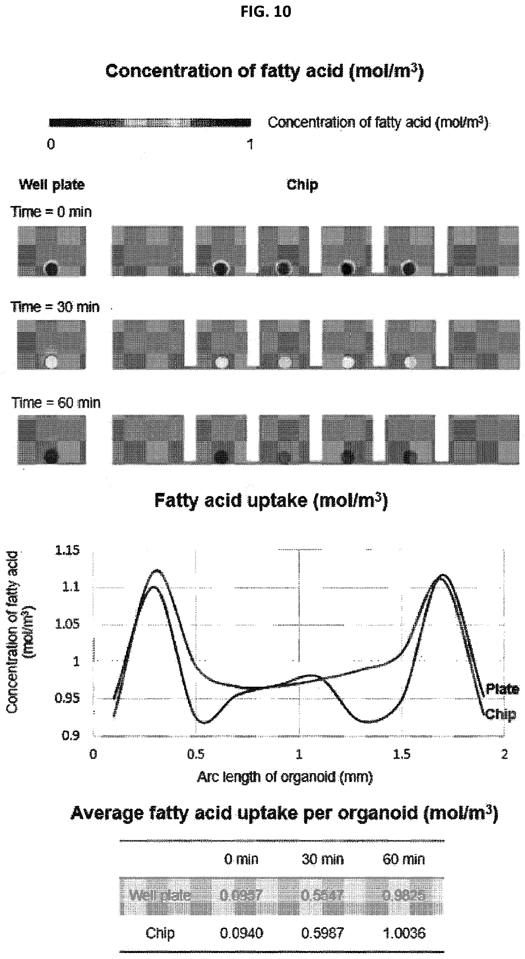

**FIG. 6** to **FIG. 10** show the results of selecting optimal culture conditions for construction of non-alcoholic steatohepatitis (NASH) organoid models. **FIG. 6A** and **FIG. 6B** show the results of tests for selecting an optimized culture platform for construction of NASH organoid models. **FIG. 7A** to **FIG. 7C** show the results of tests for selecting an optimized culture platform for construction of NASH organoid models. **FIG. 8** shows the result of test for selecting an optimized culture platform for construction of NASH organoid models. **FIG. 9** shows the result of a fluid movement simulation in a chip-based culture platform optimized to induce NASH. **FIG. 10** shows the result of a fatty acid uptake simulation in a chip-based culture platform optimized to induce NASH.

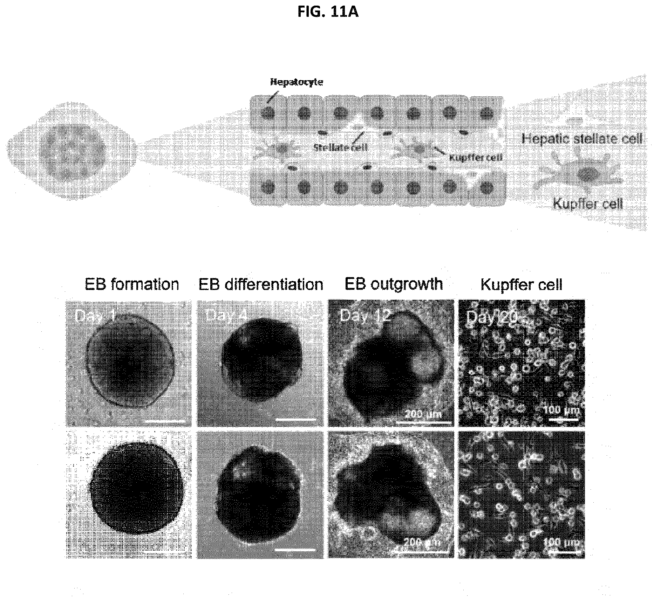

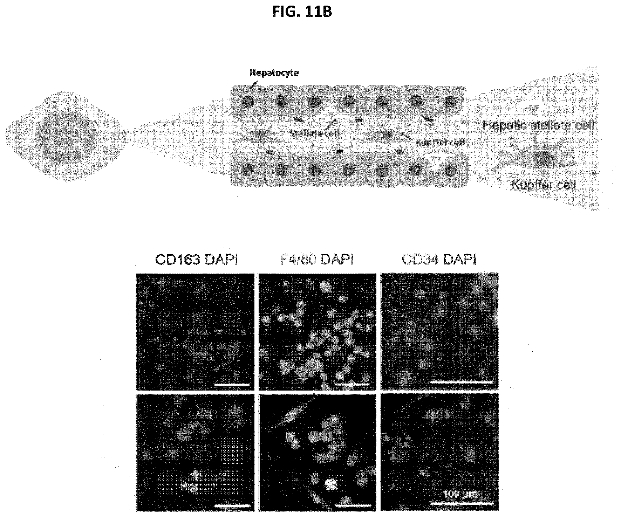

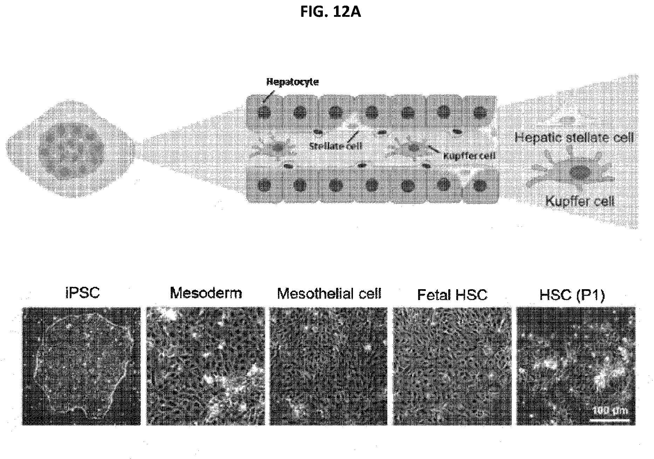

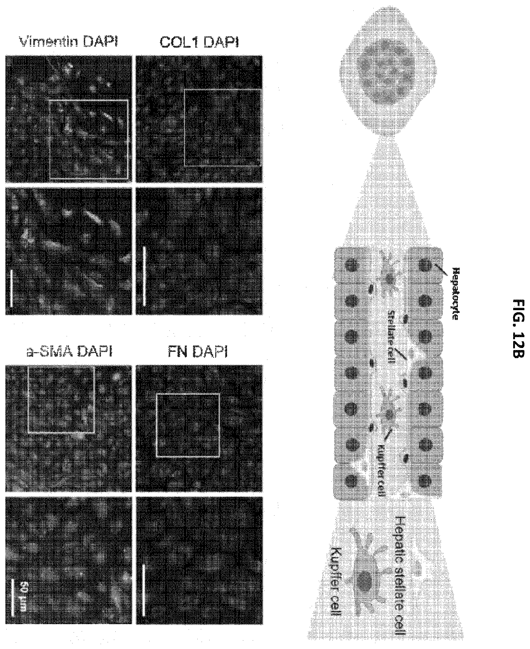

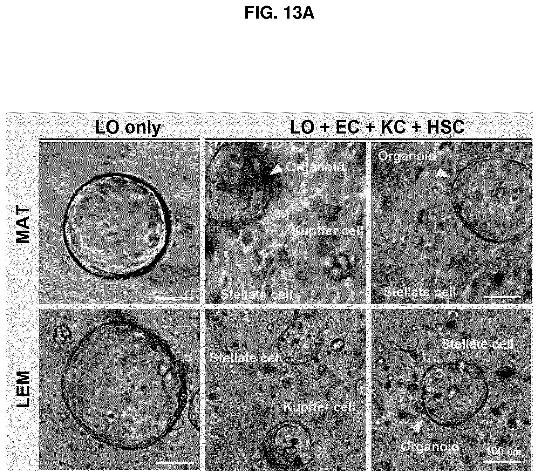

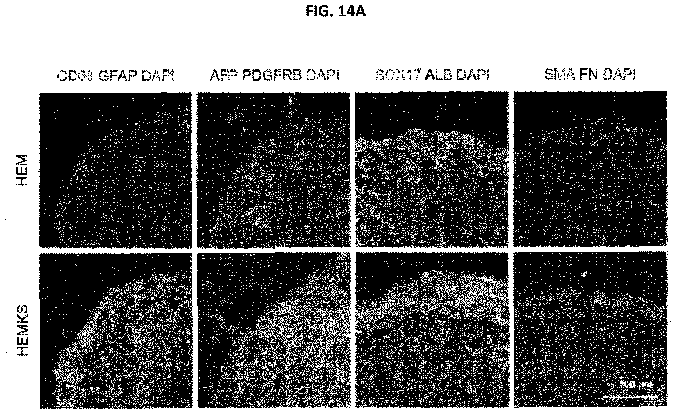

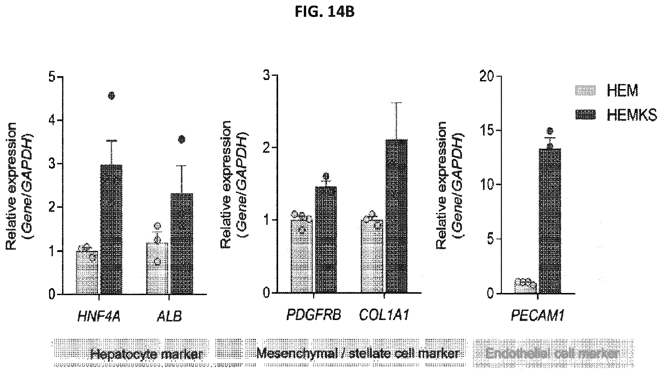

**FIG. 11** to **FIG. 14** show the results of confirming the effect of enhancing organoid differentiation through co-culture of liver tissue-specific microenvironmental cells. **FIG. 11A** and **FIG. 11B** show the results of Kupffer cell differentiation for a NASH organoid model integrated with the liver tissue microenvironment. **FIG. 12A** and **FIG. 12B** show the results of hepatic stellate cell (HSC) differentiation for a NASH organoid model integrated with the liver tissue microenvironment. **FIG. 13A** to **FIG. 13C** show the results of comparing liver differentiation and functionality of human tissue-derived liver organoids with or without co-culture of microenvironmental cells. **FIG. 14A** and **FIG. 14B** show the results of comparing liver differentiation and functionality of human iPSC-derived liver organoids with or without co-culture of microenvironmental cells.

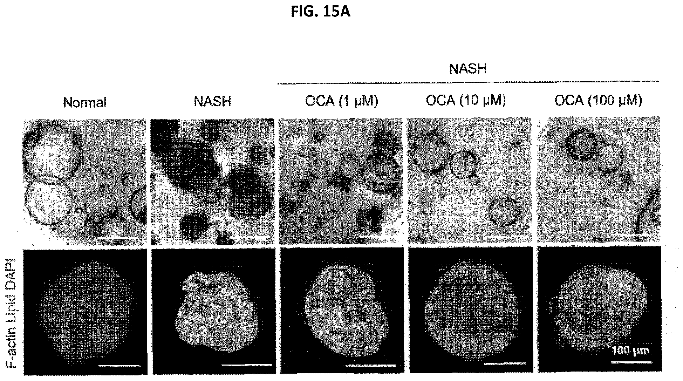

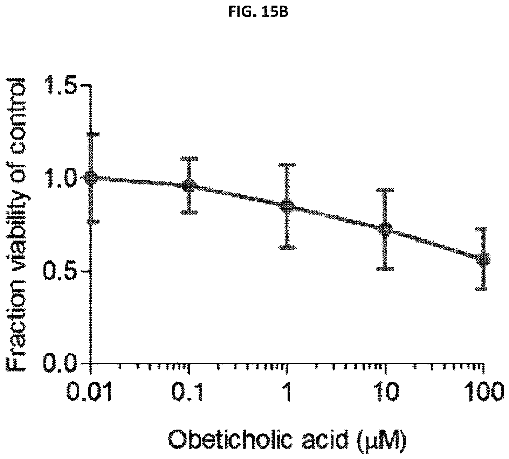

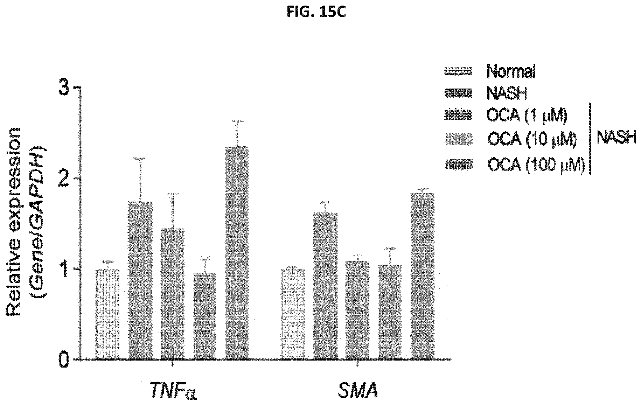

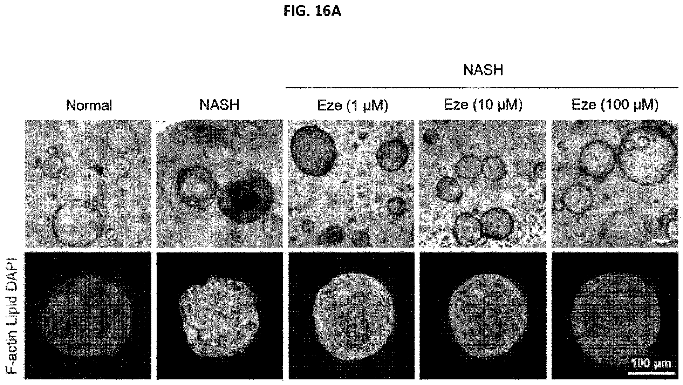

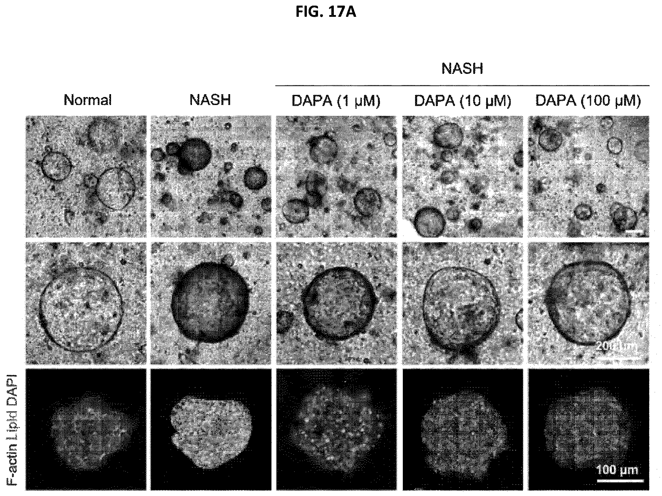

**FIG. 15** to **FIG. 17** show the results of drug efficacy evaluation using a NASH organoid model. **FIG. 15A** to **FIG. 15C** show the result of verification with obeticholic acid (OCA). **FIG. 16A** to **FIG. 16C** show the result of verification with ezetimibe (Eze). **FIG. 17A** to **FIG. 17C** show the result of verification with dapagliflozin (DAPA).

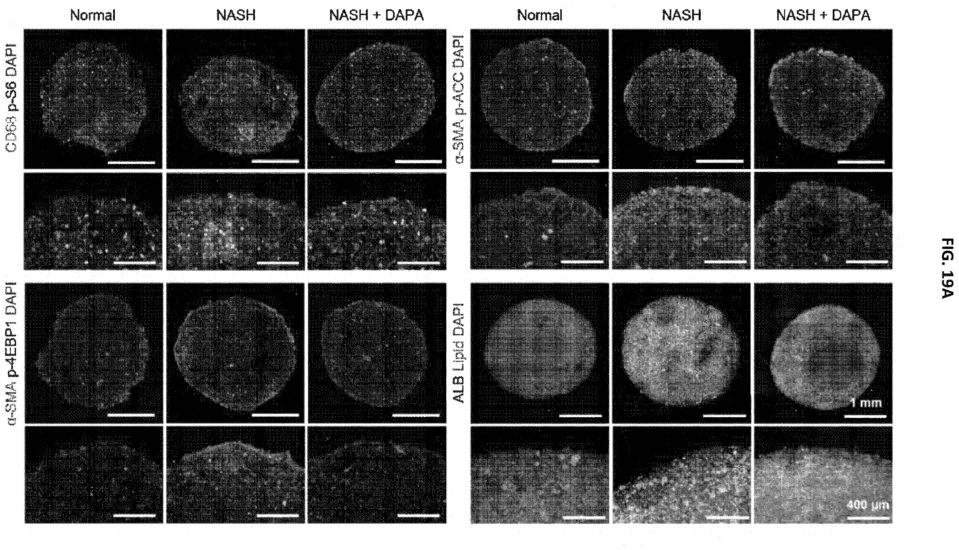

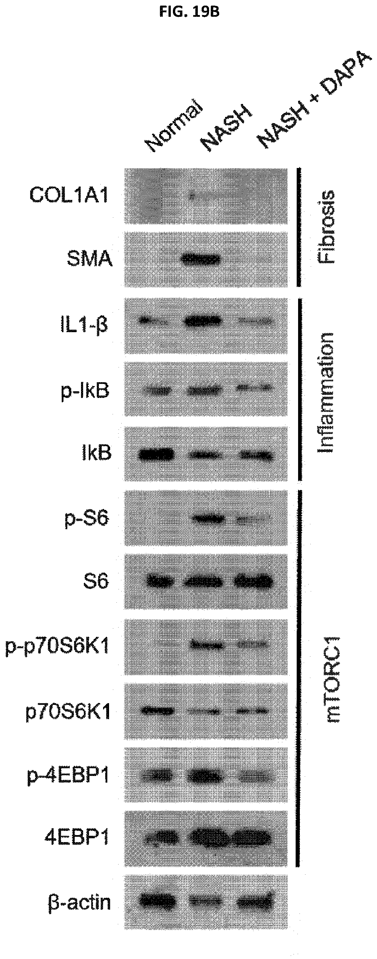

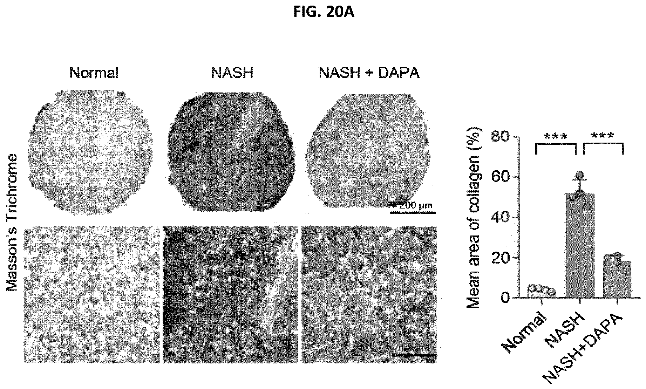

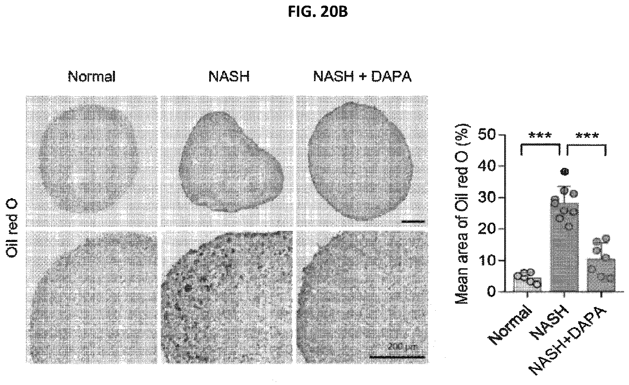

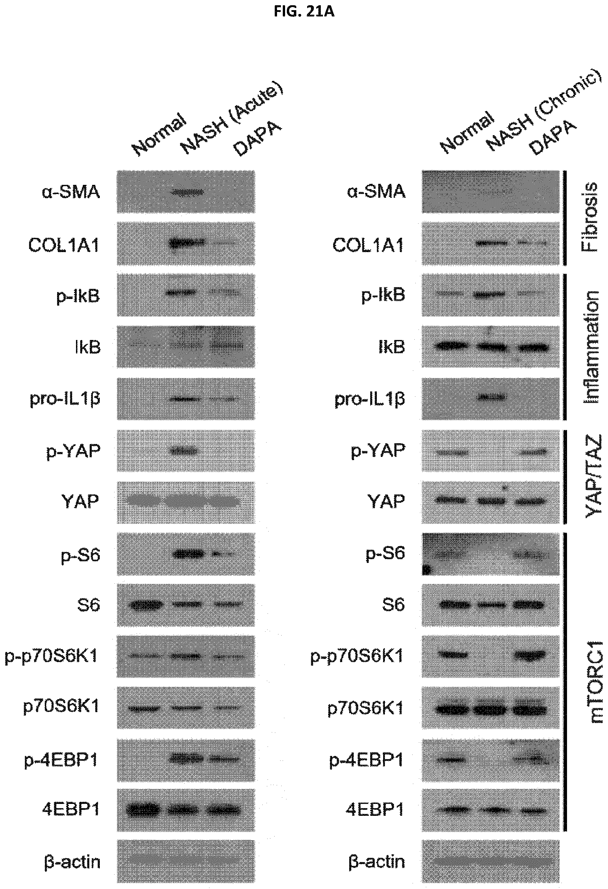

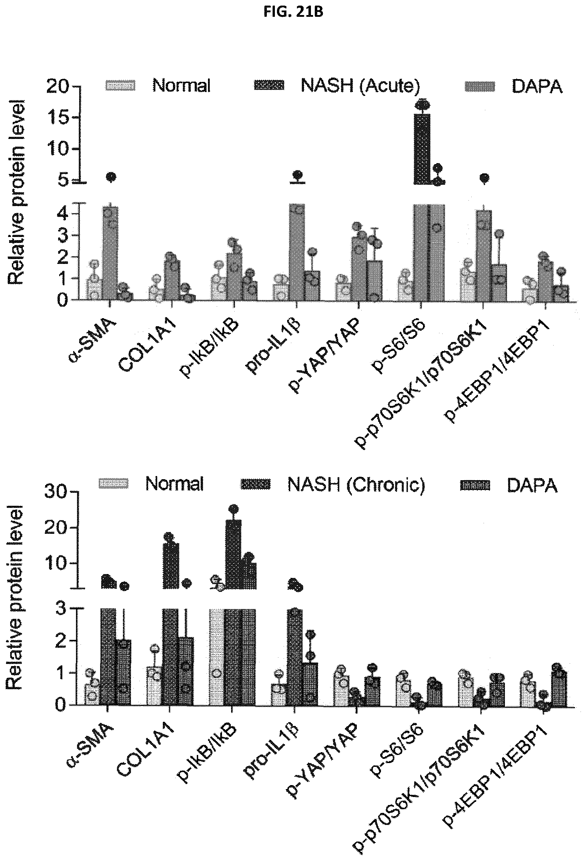

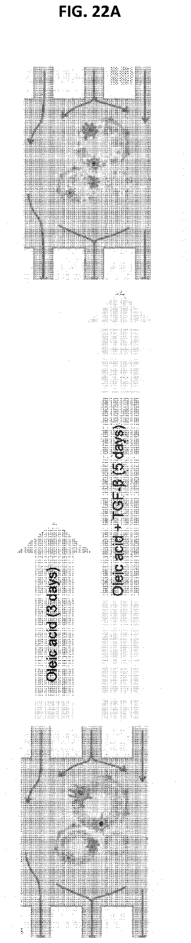

**FIG. 18** to **FIG. 22** show the results of verifying the mechanisms and effects of selected effective drugs based on NASH organoids. **FIG. 18A** and **FIG. 18B** show the results of verifying drug efficacy in a human tissue-derived liver organoid-based NASH model. **FIG. 19A** and **FIG. 19B** show the results of verifying drug efficacy in a human iPSC-derived liver organoid-based NASH model. **FIG. 20A** and **FIG. 20B** show the results of verifying drug efficacy in a human iPSC-derived liver organoid-based NASH model. **FIG. 21A** and **FIG. 21B** show the results of comparing efficacy of effective drugs and identifying treatment mechanisms by using human tissue-derived acute and chronic NASH organoid models. **FIG. 22A** to **FIG. 22C** show the results of comparing efficacy of effective drugs and identifying treatment mechanisms by using human tissue-derived acute and chronic NASH organoid models.

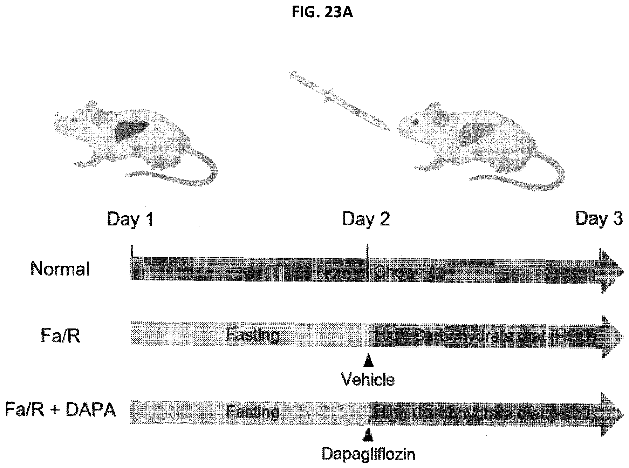

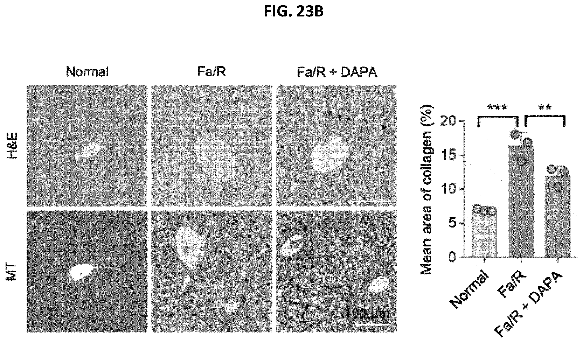

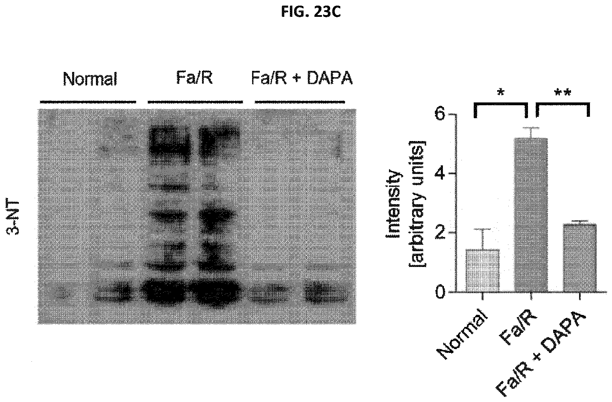

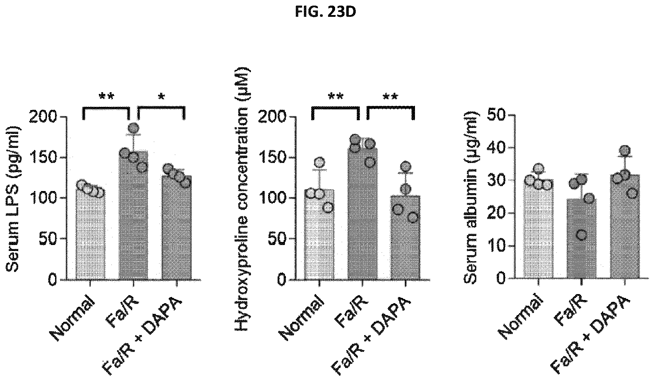

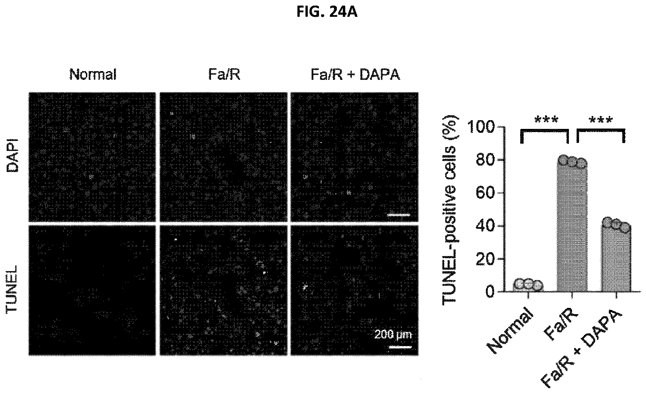

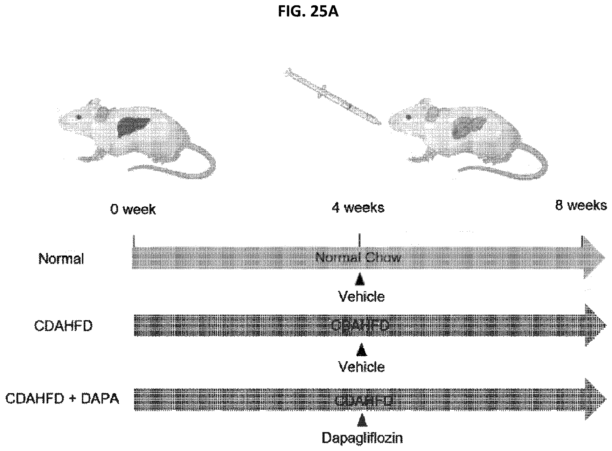

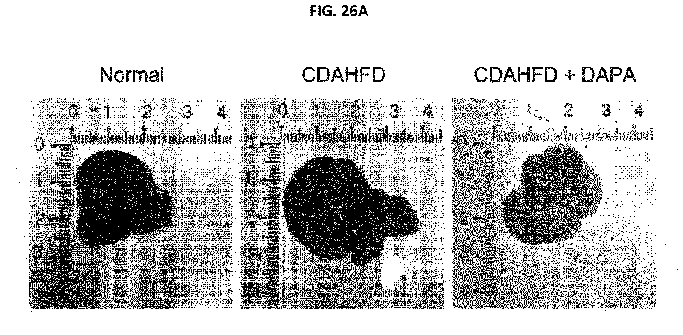

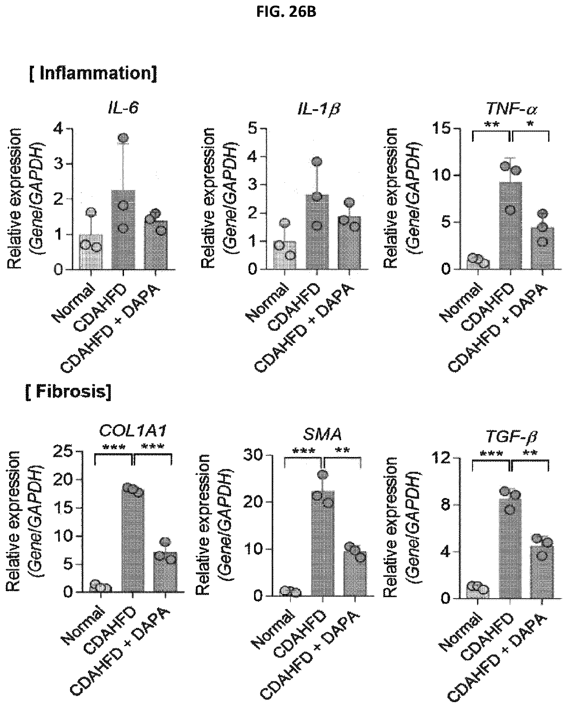

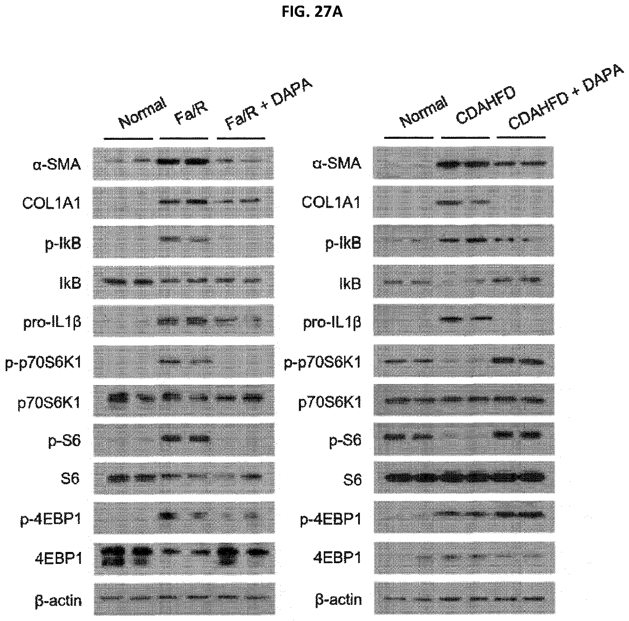

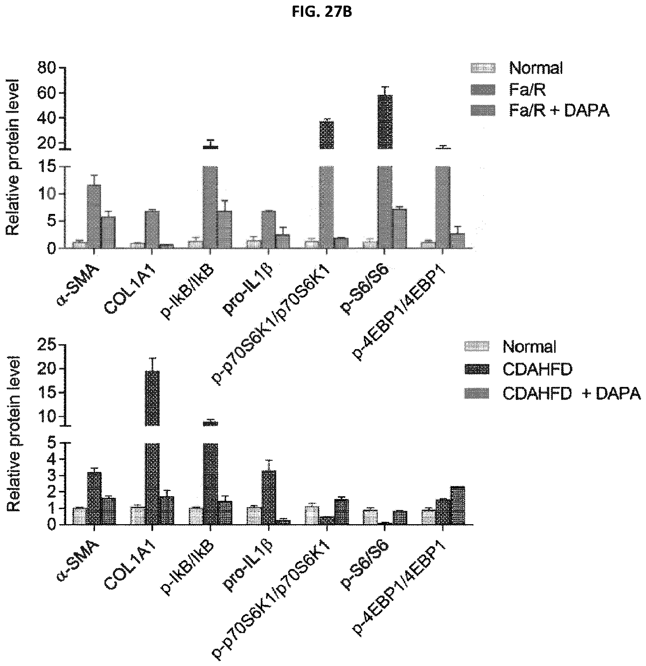

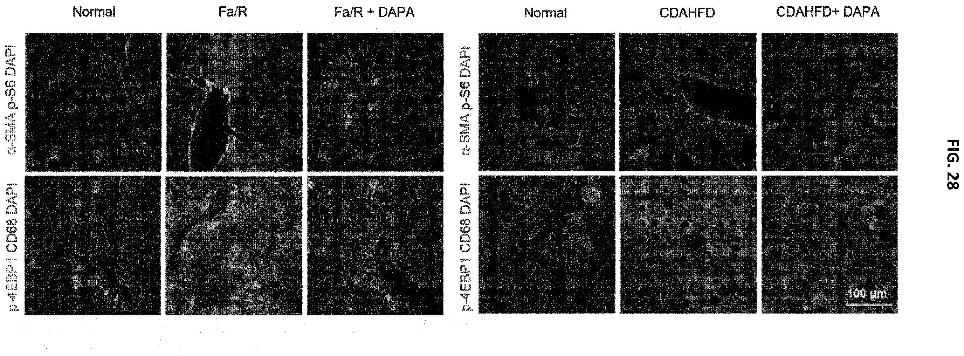

**FIG. 23** to **FIG. 28** show the results of evaluating efficacy of new therapeutic drugs discovered in NASH organoid models and verifying related mechanisms (evaluation of efficacy of drug in NASH animal models and research on mechanism). **FIG. 23A** to **FIG. 23D** show the results of verifying efficacy of DAPA in NASH animal models. **FIG. 24A** to **FIG. 24D** show the results of verifying efficacy of DAPA in NASH animal models. **FIG. 25A** to **FIG. 25D** show the results of verifying efficacy of DAPA in NASH animal models. **FIG. 26A** and **FIG. 26B** show the results of verifying efficacy of DAPA in NASH animal models. **FIG. 27A** and **FIG. 27B** show the results of comparing efficacy of effective drugs and identifying treatment mechanisms in acute and chronic NASH animal models. **FIG. 28** shows the results of comparing efficacy of effective drugs and identifying treatment mechanisms in acute and chronic NASH animal models.

### BEST MODE FOR CARRYING OUT THE INVENTION

Hereafter, the present disclosure will be described in detail with reference to the accompanying drawings. However, it is to be noted that the present disclosure is not limited to examples described herein but can be embodied in various other ways. It is to be understood that the term "comprises or includes" and/or "comprising or including" used in the document means that one or more other components, steps, operation and/or existence or addition of elements are not excluded in addition to the described components, steps, operation and/or elements unless context dictates otherwise.

Unless otherwise indicated, the practice of the disclosure involves conventional techniques commonly used in molecular biology, microbiology, protein purification, protein engineering, protein and DNA sequencing, and recombinant DNA fields, which are within the skill of the art. Such techniques are known to a person with ordinary skill in the art and are described in numerous standard texts and reference works.

Unless otherwise defined herein, all technical and scientific terms used herein have the same meaning as commonly understood by a person with ordinary skill in the art to which this disclosure belongs.

Various scientific dictionaries that include the terms included herein are well known and available to those in the art. Although any methods and materials similar or equivalent to those described herein find use in the practice or testing of the disclosure, some preferred methods and materials are described. It is to be understood that this disclosure is not limited to the particular methodology, protocols, and reagents described, as these may vary, depending upon the context in which they are used by a person with ordinary skill in the art. Hereinafter, the present disclosure will be described in more detail.

An aspect of the present disclosure provides a non-alcoholic fatty liver artificial tissue model, including: a hydrogel containing a decellularized liver tissue-derived extracellular matrix (Liver Extracellular Matrix; LEM); a liver organoid; a device including a well in which the hydrogel is located and a plurality of microchannels through which a free fatty acid flows; and a culture medium containing a free fatty acid.

In an embodiment of the present disclosure, the LEM may be a matrix in which 95% to 99.9%, more specifically 96% to 98%, and most specifically 97.18% of liver tissue cells have been removed. When decellularization is performed at a level of liver tissue cell removal out of the above-described range, the quality of the prepared scaffold composition may be degraded or the economic efficiency of the process may be reduced.

The term "extracellular matrix" refers to a protein component found in mammals and multicellular organisms and a natural scaffold for cell growth that is prepared by decellularization of tissue. The extracellular matrix can be further processed through dialysis or crosslinking.

The extracellular matrix may be a mixture of structural or non-structural biomolecules including, but not limited to, collagens, elastins, laminins, glycosaminoglycans, proteoglycans, antimicrobials, chemoattractants, cytokines and growth factors.

In mammals, the extracellular matrix may contain about 90% collagen in various forms. The extracellular matrices derived from various living tissues may differ in their overall structure and composition due to the unique role needed for each tissue.

The term "derive" or "derived" refers to a component obtained from any stated source by any useful method.

In an embodiment of the present disclosure the liver organoid may be derived from a human induced pluripotent stem cell (hiPSC) or human liver tissue.

The term "organoid" refers to an ultraminiature body organ prepared in the form of an artificial organ by culturing cells derived from tissues or pluripotent stem cells in a 3D form.

The organoid is a three-dimensional tissue analog that contains organ-specific cells which originate from stem cells and self-organize (or self-pattern) in a similar manner to the in vivo condition. The organoid can be developed into a specific tissue by patterning a restricted element (for example, a growth factor).

The organoid can have the intrinsic physiological properties of the cells and can have an anatomical structure that mimics the original state of a cell mixture (including all remaining stem cells and the neighboring physiological niche as well as limited cell types). A three-dimensional culture method allows the organoid to be better arranged in terms of cell to cell functions and to have an organ-like form with functionality and a tissue-specific function.

Specifically, when the liver organoid is derived from the human induced pluripotent stem cell (hiPSC), the liver organoid may be cultured on the hydrogel. In this case, a hydrogel is injected into a well to form a hydrogel bed on which an organoid is to be cultured. When cells are injected into the hydrogel bed, an organoid having a three-dimensional structure is formed within 24 hours. In this case, the organoid can be cultured while being supported by a matrix and receiving a microfluidic flow of the culture medium. In particular, since the organoid is directly exposed to the culture medium, the efficiency in transferring the culture medium into the organoid is very excellent. Thus, an organoid having a three-dimensional structure can be formed through culture of the liver organoid on the hydrogel.

Alternatively, when the liver organoid is derived from the human liver tissue, the liver organoid may be cultured within the hydrogel. In this case, the hydrogel condenses over time to form a hard matrix, and the hydrogel containing the organoid remains attached to the bottom of the well during the culture process, and, thus, the organoid can be cultured while continuously receiving a microfluidic flow of the culture medium.

The device includes a well in which the hydrogel is located and a plurality of microchannels through which a free fatty acid flows. The device may be manufactured using a known material, specifically a PDMS polymer. Also, the device may be composed of a plurality of chambers. Specifically, the device is equipped with 8 chip units connected to media, and each unit is composed of 4 culture chambers having D of 7.10 mm. Further, each chamber has a well having D of 5 mm, and 8.66 mm wide media chambers are located at respective ends of each unit. When a device is manufactured as described above, it has the same sizes as a 96-well plate and can be used as a high-throughput multiwell device.

Also, when the liver organoid is derived from the human induced pluripotent stem cell (hiPSC), the well in which the hydrogel is located may have a depth of 2 mm to 4 mm, specifically 3 mm. When the liver organoid is derived from the human liver tissue, the well in which the hydrogel is located may have a depth of 0.5 mm to 1.5 mm, specifically 1 mm. If the well is fabricated to have a depth out of the above range, the flow of the culture medium through the channel may not be properly delivered to the organoid within the hydrogel.

In an embodiment of the present disclosure, the free fatty acid may have a concentration of 100 µM to 900 µM, specifically 200 µM to 800 µM, and most specifically 800 µM. The free fatty acid flows through the plurality of microchannels provided in the above-described device to the well in which the hydrogel is located, and affects the liver organoid on or within the hydrogel. If the free fatty acid is used at a concentration out of the above range, the characteristics of non-alcoholic fatty liver may not appear or the cells in the tissue model may die.

Also, the culture medium containing the free fatty acid induces a non-alcoholic fatty liver disease by continuously exposing the liver organoid to the free fatty acid contained in the culture medium while culturing the liver organoid. The components of the culture medium may be a mixture of known substances used for liver organoid culture in addition to free fatty acids.

In an embodiment of the present disclosure, the free fatty acid may be any one selected from oleic acid, palmitic acid and linoleic acid, and may be specifically oleic acid.

In an embodiment of the present disclosure, the liver organoid may be co-cultured with any one or more of vascular cells, mesenchymal stem cells, Kupffer cells (KC), and hepatic stellate cells (HSC), specifically Kupffer cells (KC) and hepatic stellate cells (HSC). The Kupffer cells are immune cells present in the liver, and secrete cytokines and reactive oxygen species (ROS) when inflammatory Kupffer cells are activated through induction of non-alcoholic fatty liver. Further, the hepatic stellate cells function to secrete fibrotic factors in a non-alcoholic fatty liver disease. The non-alcoholic fatty liver artificial tissue model of the present disclosure further includes any one or more of the above-described Kupffer cells and hepatic stellate cells in addition to the liver organoid and thus can further specifically reflect the microenvironment of the liver. Accordingly, it is possible to fabricate a non-alcoholic fatty liver with a high degree of mimicry.

Another aspect of the present disclosure provides a method of fabricating a non-alcoholic fatty liver artificial tissue model, including: a process of fabricating the non-alcoholic fatty liver artificial tissue model; and a process of perfusing the non-alcoholic fatty liver artificial tissue model with a culture medium containing a free fatty acid.

The process of fabricating the non-alcoholic fatty liver artificial tissue model is to fabricate the non-alcoholic fatty liver artificial tissue model. Specifically, it may be composed of a process of fabricating a device (microfluidic chip) including a well in which a hydrogel is located and a plurality of microchannels through which a free fatty acid flows by using a PDMS polymer; and a process of locating the hydrogel and the liver organoid in the well. Details of the hydrogel, liver organoid, and device are the same as those of the non-alcoholic fatty liver artificial tissue model described above.

The process of perfusing is to perfuse the non-alcoholic fatty liver artificial tissue model with the culture medium containing the free fatty acid. As described above, the culture medium containing the free fatty acid flows through the microchannels of the device to the well in which the hydrogel and the liver organoid are located, and the liver organoid is continuously exposed to the free fatty acid and thus exhibits the phenotypes of non-alcoholic fatty liver.

In an embodiment of the present disclosure the process of perfusing may be performed by shaking the non-alcoholic fatty liver artificial tissue model on a rocking shaker. The perfusion of the culture medium containing the free fatty acid may be performed by a known device, and the use of a rocking shaker with high accessibility and operational convenience can make a continuous flow of the culture medium to the liver organoid. Specifically, when the non-alcoholic fatty liver artificial tissue model is placed on the rocking shaker and filled with the culture medium and the rocking shaker is operated, the culture medium flows and perfusion occurs as the rocking shaker is tilted from side to side.

Yet another aspect of the present disclosure provides a method of screening a therapeutic drug for a non-alcoholic fatty liver disease, including: a process of treating the non-alcoholic fatty liver artificial tissue model with a candidate substance; and a process of comparing a group treated with the candidate substance and a control group.

The process of treating with the candidate substance is to treat the non-alcoholic fatty liver artificial tissue model with the candidate substance, and the treatment with the candidate substance may vary depending on the intended route of administration and dosage of the candidate substance.

Further, the process of comparing the group treated with the candidate substance and the control group may be to compare the non-alcoholic fatty liver artificial tissue model treated with the candidate substance and the control group. The control group may be a non-alcoholic fatty liver artificial tissue model treated with or not treated with a conventionally known non-alcoholic fatty liver therapeutic drug or a known substance within the range that does not inhibit or increase the physiological activity of the liver organoid in the non-alcoholic fatty liver model.

The group treated with the candidate substance may be compared to the control group by analyzing the levels of fat accumulation in the liver organoids, the viability of the liver organoids and the differentiation and functionality of the liver organoids and/or by checking various indicators in the liver organoids or culture media.

The method of screening may further include a process of selecting a non-alcoholic fatty liver therapeutic drug. The process of selecting may be to select a non-alcoholic fatty liver therapeutic drug when a reduction of fat accumulation in the liver organoids, an increase in viability of the liver organoids, the differentiation of the liver organoids, the recovery of functionality, and/or an increase in improved indicators in the liver organoids or culture media in the above-described process of comparing. If a conventionally known non-alcoholic fatty liver therapeutic drug is used as a control group, any alcoholic fatty liver therapeutic drug can be determined and selected as having an improved effect compared to the conventionally known non-alcoholic fatty liver therapeutic drug when it shows an improved effect compared to the control group.

### MODE FOR CARRYING OUT THE INVENTION

Hereinafter, one or more specific embodiments will be described in more detail through examples. However, these examples are for illustrative purposes of one or more embodiments, and the scope of the present disclosure is not limited to these examples.

### Example 1: Fabrication of non-alcoholic fatty liver artificial tissue model

### Example 1-1. Fabrication of decellularized liver tissue-derived extracellular matrix

A porcine liver tissue was isolated and sliced, and the liver tissue was stirred with 1% Triton X-100 and 0.1% ammonium hydroxide to remove only the cells of the liver tissue and prepare a decellularized liver tissue. Thereafter, the decellularized liver tissue was lyophilized and ground to fabricate a decellularized liver tissue-derived extracellular matrix (Liver Extracellular Matrix; LEM).

10 mg of the LEM was dissolved in a 4 mg/ml pepsin solution (a solution of 4 mg of pepsin powder in 1 ml of 0.02 M HCl) for 48 hours. Gelation was performed at a temperature of 37°C for 30 minutes after setting a neutral pH in an electrolyte state of 1×PBS buffer by using 10×PBS and 1 M NaOH with uniform blending to fabricate a scaffold composition in the form of a hydrogel.

The fabricated LEM was subjected to H&E staining before and after the decellularization process to confirm that the structure of the fabricated decellularized matrix was well maintained and all cellular components were removed. Also, it was confirmed through scanning electron microscopy that an internal structure in the form of a fiber bundle formed a stable polymer network. Further, it was confirmed that most of the cellular components were removed and glycosaminoglycans (GAGs) were well preserved through quantitative comparison of DNAs and quantitative analysis of GAGs, one of the representative extracellular matrix components **(****FIG. 1A****).**

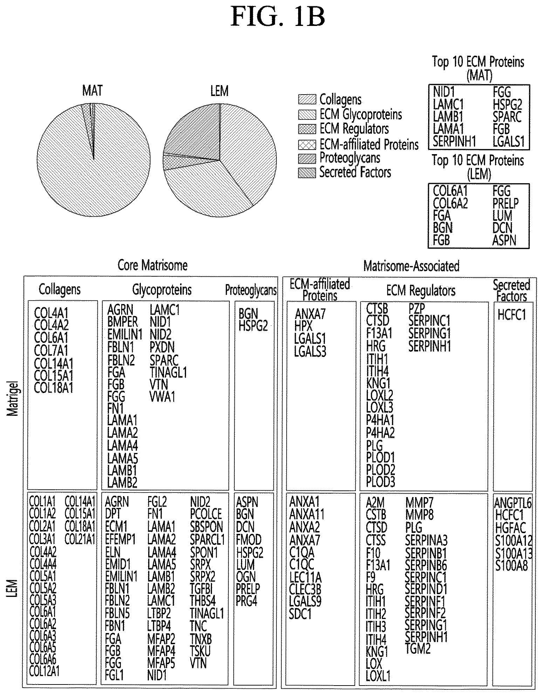

Furthermore, proteomic analysis was performed to identify the components of the LEM. Thus, it was confirmed that various extracellular matrices (collagens, glycoproteins, proteoglycans) and growth factor proteins specific to liver tissue are contained in the LEM. Also, it was confirmed that Matrigel (MAT), a commercially available scaffold, is mostly composed of ECM glycoproteins, whereas the LEM is mostly composed of collagens and ECM glycoproteins, as well as other components including proteoglycans and ECM regulators. Among top ten extracellular matrix (ECM) proteins detected the LEM, biglycan (BGN), lumican (LUM) and asporin (ASPN), which are specifically present in the LEM, are major proteins involved in ECM remodeling during liver tissue development, and PRELP is known as a protein that is important in maintaining the structure of a normal hepatocyte. Accordingly, it was confirmed that the fabricated LEM scaffold contains various extracellular matrix proteins present in the liver tissue and important in the structure, development and function of liver compared to Matrigel **(****FIG. 1B****).**

### Example 1-2. Fabrication of device for non-alcoholic fatty liver artificial tissue model

A device fabricated in the form of a microfluidic chip by using a PDMS polymer is equipped with 8 chip units connected to media, and each unit is composed of 4 culture chambers having D of 7.10 mm. Further, each chamber has a well having D of 5 mm (manufactured in two different depths, H = 3 mm and 1 mm), and 8.66 mm wide media chambers are located at respective ends of each unit **(****FIG. 2A****).**

Specifically, if the device is fabricated in the form of a developed 32-multiwell device, it is possible to more efficiently induce steatohepatitis and also possible to perform high-throughput drug screening of disease organoids. The high-throughput multiwell device has the same sizes as a conventional 96-well plate and thus is compatible with a general-purpose 96-well based analysis equipment including a plate reader **(****FIG. 2B****).**

When the liver organoid was derived from a human induced pluripotent stem cell (hiPSC), the liver organoid was cultured on the extracellular matrix located in the well having a depth of 3 mm, and when the liver organoid was derived from a human tissue, the liver organoid was cultured within the extracellular matrix located in the well having a depth of 1 mm **(****FIG. 3A****).**

Moreover, the device was fabricated to enable effective formation of microfluidic flow by using a commercially available rocking shaker **(****FIG. 3B****).**

### Test Example 1: Confirmation of liver organoid culture in device of the present disclosure and optimization of non-alcoholic fatty liver model

### Test Example 1-1. Confirmation of liver organoid culture in device of the present disclosure

Liver organoids were cultured based on the LEM in the above-described device and compared to a conventional well plate in a static culture environment. Specifically, the liver organoids derived from a human liver tissue were prepared and analyzed on day 7 of culture.

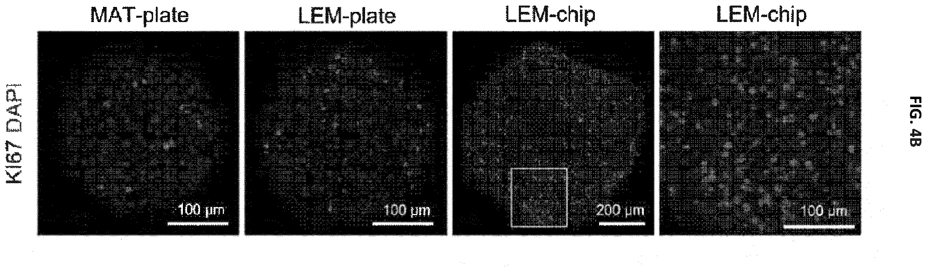

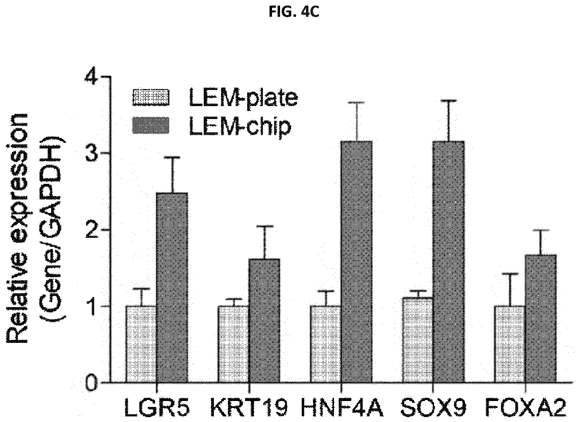

As can be seen from **FIG. 4****,** it was confirmed that the size of the liver organoid (LEM-chip) cultured in the device of the present disclosure significantly increased compared to the liver organoid (LEM-plate) cultured on the conventional well plate. As a result of quantitative analysis of the organoid size, a significant increase in size was observed in the liver organoid group cultured while receiving a flow of the culture medium in the device **(****FIG. 4A****).**

Also, as a result of immunostaining of KI67, a proliferation-related marker, to verify the above result, it was confirmed that the Matrigel group as a control group cultured on the plate and the LEM group showed similar proliferation ability, but the expression of KI67 significantly increased in the group in which the LEM and the microfluidic device were combined **(****FIG. 4B****).** Accordingly, it is assumed that the proliferation ability of the liver organoid where oxygen and nutrients were supplied efficiently was greatly increased, and, thus, the size of organoid increased.

Further, when gene expression was compared by quantitative PCR, the expression levels of LGRS, KRT19, HNF4A, SOX9, and FOXA2 increased in the liver organoid cultured in the device of the present disclosure compared to the liver organoid prepared in a static plate culture environment **(****FIG. 4C****).**

### Test Example 1-2. Optimization of treatment concentration of free fatty acid

Human liver organoids were cultured in an LEM-multiwell microfluidic device platform (LEM-Chip) with the highest steatohepatitis organoid-inducing efficiency and treated with a free fatty acid (oleic acid) at different concentrations, respectively, to test an optimal concentration for disease model construction.

As a result, when the human tissue-derived liver organoids were cultured on a platform in which a decellularized LEM hydrogel and the multiwell device were combined, and treated with a fatty acid at different concentrations, respectively, it was confirmed that fat accumulated inside the liver organoids at different concentrations, causing inflammation as well as damage and deformation of the liver organoid-specific morphology and structure **(****FIG. 5A****).**

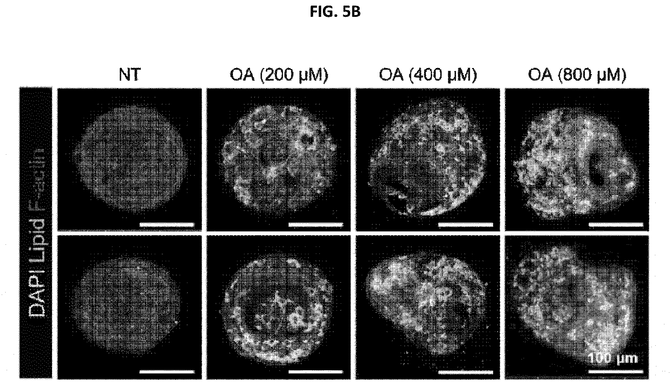

As a result of immunostaining to identify the amount of fat accumulation inside the liver organoid, it was confirmed that the amount of fat accumulated inside the organoid increased as the concentration of the fatty acid used for treatment increased. Therefore, it was confirmed that a non-alcoholic fatty liver organoid model can be implemented by the decellularized LEM-multiwell device platform **(****FIG. 5B****).**

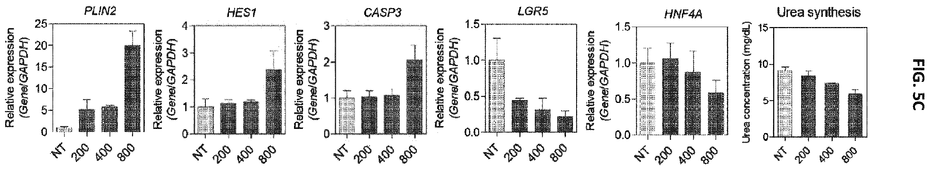

As a result of analyzing the gene expression level for each treatment concentration of the fatty acid through quantitative PCR, it was confirmed that the characteristics of steatohepatitis organoids were maximally expressed at a high treatment concentration of 800 µM. Further, as a result of comparing the urea synthesis ability to analyze the hepatic functionality, it was confirmed that the urea synthesis ability decreased as the treatment concentration of the fatty acid increased **(****FIG. 5C****).**

* PLIN2 : Lipid droplet marker, HES1 : NASH/Fibrosis-related notch signaling marker, CASP3 : Cell death marker, LGRS : Stemness marker, HNF4A : Mature hepatocyte marker

### Test Example 2: Selection of optimal culture condition for construction of non-alcoholic steatohepatitis (NASH) organoid model

### Test Example 2-1. Selection of optimized culture platform for construction of non-alcoholic steatohepatitis (NASH) organoid model (1)

To select an optimal culture platform for construction of a non-alcoholic steatohepatitis organoid model, human liver organoids were fabricated in three different culture conditions: (1) Matrigel (MAT), which is a commercially available culture scaffold; (2) a decellularized liver tissue scaffold (LEM); and (3) a system (LEM-Chip) in which a decellularization liver tissue scaffold and a multiwell device are combined, and then treated with 800 µM oleic acid to induce a fatty liver model. The liver organoids were prepared by using adult liver stem cells extracted from a human liver tissue.

As a result, it was confirmed that the liver organoids cultured in the decellularized LEM hydrogel scaffold were similar in shape and size to the liver organoids cultured in the Matrigel group (MAT), and the liver organoids having a larger size were formed due to efficient oxygen supply by the effect of a microfluidic flow of the culture medium through the channels in the culture environment combined with the multiwell device. Even when the fatty liver model was induced through treatment with the fatty acid, it was confirmed that fat accumulation and inflammation inside the organoid were most readily induced in the LEM-chip group **(****FIG. 6A****).**

Further, as a result of quantitative PCR on the respective liver organoids treated with the fatty acid for 3 days to evaluate the NASH-inducing efficiency depending on the culture condition, it was confirmed that NASH was more readily induced in the decellularized liver tissue scaffold-multiwell device group (LEM-Chip) than in the Matrigel group (MAT) as a control group and the decellularized liver tissue scaffold group (LEM) **(****FIG. 6B****).** (TNF-α: inflammatory marker, SMA: fatty liver/fibrosis marker, PLIN2: fat accumulation marker, ALB: mature hepatic differentiation marker)

Accordingly, the liver organoid culture platform in which the decellularized liver tissue-derived scaffold and the multiwell device were combined was selected as an optimal platform and used later for NASH-inducing tests.

### Test Example 2-2. Selection of optimized culture platform for construction of non-alcoholic steatohepatitis (NASH) organoid model (2)

To select an optimal culture platform for construction of a non-alcoholic steatohepatitis organoid model, human liver organoids were fabricated in three different culture conditions: (1) Matrigel (MAT), which is a commercially available culture scaffold; (2) a decellularized liver tissue scaffold (LEM); and (3) a system (LEM-Chip) in which a decellularization liver tissue scaffold and a multiwell device are combined, and then treated with 800 µM oleic acid to induce a fatty liver model. The liver organoids were prepared by using adult liver stem cells extracted from a human liver tissue.

As a result, when steatohepatitis was induced for 3 days in the above-described three culture conditions and the steatohepatitis-inducing efficiency was compared through immunostaining, the expression levels of SMA and VIM, liver fibrosis markers, were observed much higher in the organoids cultured in the culture environment combined with the multiwell device than in the liver organoids cultured in MAT under the conventional static culture environment (well plate) and LEM **(****FIG. 7A****).**

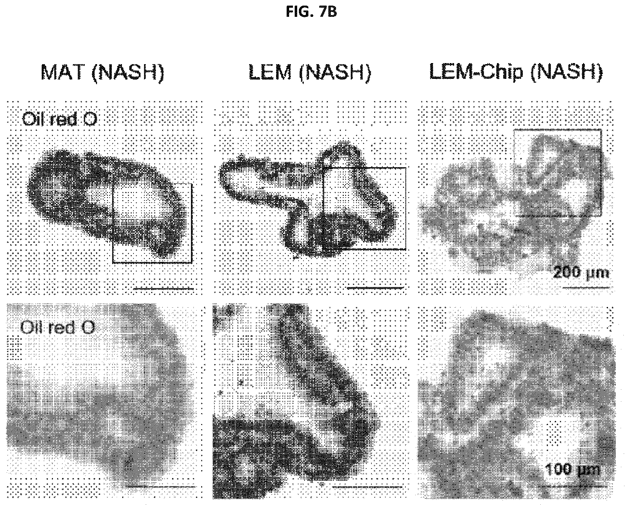

Further, as a result of Oil red O staining (intracellular fat staining) to evaluate the NASH-inducing efficiency depending on the culture condition, it was confirmed that fatty acid accumulation inside the liver organoid was more readily induced in the decellularized liver tissue scaffold-multiwell device group (LEM-Chip) than in the Matrigel group (MAT) as a control group under the static culture environment and the decellularized liver tissue scaffold group (LEM) **(****FIG.** 7B).

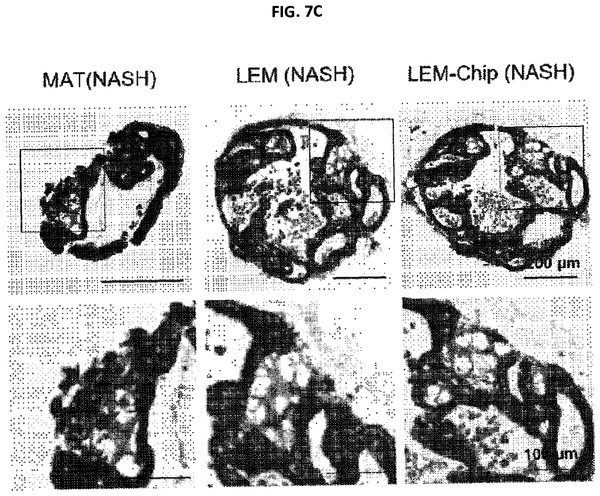

Furthermore, as a result of checking collagen accumulated inside the organoid through Masson's Trichrome (MT) staining, it was confirmed that the amount of collagen accumulation was higher in the LEM-Chip group than in the MAT and LEM groups. Accordingly, it was confirmed that the fatty acid used for treatment using the microfluidic chip in the culture condition, compared to the static culture condition, was most readily induced to accumulate in the liver organoid by the effect of a microflow through the microchannels and fibrosis was most readily induced **(****FIG. 7C****).**

Accordingly, the liver organoid culture platform in which the decellularized liver tissue-derived scaffold and the multiwell device were combined was selected as an optimal platform and used later for NASH-inducing tests.

### Test Example 2-3. Selection of optimized culture platform for construction of non-alcoholic steatohepatitis (NASH) organoid model (3)

When non-alcoholic steatohepatitis (NASH) was induced in the liver organoids, an inflammatory cytokine immuno-antibody array was performed to check whether the amount of secretion and diversity of inflammatory cytokines increases in the LEM-Chip condition selected as the optimal group, compared to the other culture conditions. The human tissue-derived liver organoids were cultured in a normal group and a NASH-induced group prepared in MAT as a control group, and NASH-induced groups prepared in the LEM culture condition and the LEM-Chip condition, respectively, and then analyzed.

When an immuno-antibody array was performed on 40 types of inflammatory cytokines, quantitative analysis was performed on 7 major inflammatory cytokines detected in higher amounts of secretion. As a result, seven cytokines (MIP-1δ, sTNFRI, RANTES, TIMP-2, IL-11, MCP-1, IL-8) tended to increase in the NASH-induced groups compared to the Normal group. Especially, it was confirmed that the highest amounts of inflammatory cytokine secretion were detected in the NASH organoid group cultured in the LEM-Chip **(****FIG. 8****).** Accordingly, it was confirmed that compared to the conventional culture platform, the culture platform in which the LEM scaffold and the microfluidic chip were combined according to the present disclosure is the most optimized platform for inducing a NASH model.

### Test Example 2-4. Simulation of fluid movement in chip-based culture platform optimized for induction of non-alcoholic steatohepatitis (NASH)

A prediction simulation was performed for fluid movement within the chip for accelerating fatty acid uptake into the organoid in the chip condition with a flow of the culture medium when non-alcoholic steatohepatitis (NASH) is induced by treating the culture medium with the free fatty acid rather than when NASH is induced in a conventional static plate condition without a flow of the culture medium.

The flow of fluid over time was predicted by performing a fluid simulation in the chip condition prepared to induce non-alcoholic steatohepatitis. It was confirmed that since the speed and direction of a fluid change depending on the movement of the rocking shaker, flows of the fluid formed by the rocking shaker can be more diverse than in the conventional static plate culture environment by adjusting the speed and angle of the rocking shaker **(****FIG. 9****).** Accordingly, it can be predicted that the free fatty acid contained in the culture medium can be more efficiently transferred into the organoid in a dynamic chip environment with a flow of the culture medium than in a plate environment without any fluid flow.

### Test Example 2-5. Simulation of fatty acid uptake in chip-based culture platform optimized for induction of non-alcoholic steatohepatitis (NASH)

**A** fatty acid uptake simulation was performed in each condition to check whether a fatty acid is more efficiently absorbed into the organoid in the chip condition with a flow of the culture medium when non-alcoholic steatohepatitis (NASH) is induced by treating the culture medium with the free fatty acid rather than when NASH is induced in a conventional static plate condition without a flow of the culture medium.

Fluid flow and mass transfer simulations for a conventional static well plate condition and a dynamic chip condition were performed to predict fatty acid uptake of the organoid for 60 minutes. As a result, it was confirmed that in the dynamic chip culture system, the movement and exchange of the free fatty acid contained in the culture medium is promoted through the flow of the fluid, which causes a rapid increase in fatty acid uptake into the organoid compared to the well plate condition, which is a static culture system **(****FIG. 10****).**

### Test Example 3: Confirmation of organoid differentiation promoting effect through co-culture of liver tissue-specific microenvironmental cell

### Test Example 3-1. Differentiation of Kupffer cell for non-alcoholic steatohepatitis organoid model integrated with liver tissue microenvironment

Kupffer cells are immune cells present in the liver and are known to play an important role in disease phenotypes, such as secreting cytokines and reactive oxygen species (ROS) when inflammatory Kupffer cells are activated in the induction of non-alcoholic fatty liver. Therefore, the Kupffer cells that can be co-cultured with liver organoids were differentiated from human induced pluripotent stem cells to fabricate a NASH model equipped with the liver tissue microenvironment.

As a result, it was confirmed that embryoid bodies (EBs) were prepared from the human induced pluripotent stem cells and then differentiated through macrophage precursors into the Kupffer cells **(****FIG. 11A****).**

As a result of immunostaining to analyze protein marker expression of the prepared Kupffer cells, it was confirmed that F4/80 and CD163, monocyte and M2 immune cell markers, and CD34, a Kupffer cell-related marker, were well expressed **(****FIG. 11B****).**

### Test Example 3-2. Differentiation of hepatic stellate cell (HSC) for non-alcoholic steatohepatitis organoid model integrated with liver tissue microenvironment

In the expression of non-alcoholic fatty liver inflammation, it is known that hepatic stellate cells play a role in secreting important fibrotic factors as they are activated. Therefore, to fabricate a NASH model equipped with the liver tissue microenvironment, the hepatic stellate cells that can be co-cultured with liver organoids were differentiated from human induced pluripotent stem cells.

As a result, it was confirmed that the hepatic stellate cells were differentiated from the human induced pluripotent stem cells through mesoderm and mesothelial cells and can be subcultured **(****FIG. 12A****).**

Also, as a result of immunostaining on day 14 after differentiation of hepatic stellate cells to check marker expression, it was confirmed that COL1 and FN, extracellular matrix (ECM) markers secreted by hepatic stellate cells, and vimentin and a-SMA, hepatic stellate cell differentiation markers, were well expressed **(****FIG. 12B****).**

### Test Example 3-3. Comparison of liver differentiation and functionality of human tissue-derived liver organoid with or without co-culture of microenvironmental cell

To check whether a liver organoid improves a hepatic differentiation marker and functionality when vascular endothelial cells (EC), Kupffer cells (KC), and hepatic stellate cells (HSC) present in the liver are co-cultured with human tissue-derived normal liver organoid (LO), these cells were contained in liver organoids cultured in Matrigel (MAT) and liver organoids cultured in the decellularized liver tissue-derived scaffold (LEM) and cultured together. A group in which the microenvironmental cells were not co-cultured was used as a control group. Human umbilical vein endothelial cells (HUVECs) were used as vascular endothelial cells, and cells differentiated from human induced pluripotent stem cells (iPSCs) were used as Kupffer cells and hepatic stellate cells. After 25,000 vascular endothelial cells, 25,000 Kupffer cells, and 20,000 hepatic stellate cells were added to 30 mL of MAT or LEM hydrogel in which the liver organoids were cultured and co-cultured, quantitative PCR and urea synthesis ability analysis were performed 3 days later.

When vascular cells, hepatic stellate cells, and Kupffer cells were co-cultured in human liver organoids cultured in Matrigel as a control group and the decellularized LEM hydrogel scaffold, it was confirmed that microenvironmental cells were distributed around the liver organoids in the both groups and co-cultured **(****FIG. 13A****).**

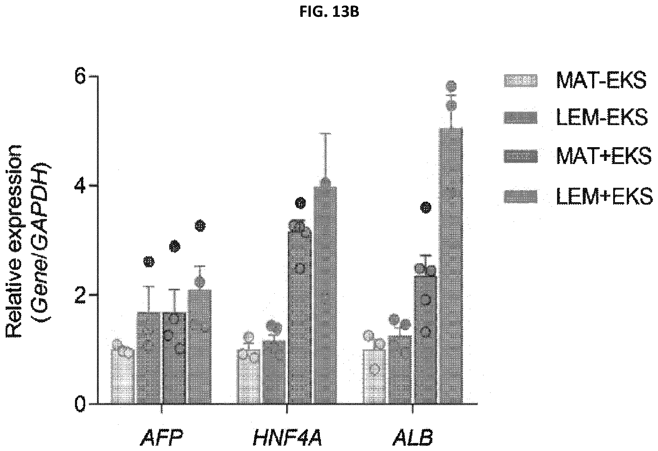

Further, as a result of quantitative PCR to compare hepatic differentiation marker expression, it was confirmed that the expression of hepatic differentiation markers in the liver organoids cultured in the LEM hydrogel was superior to that of the Matrigel group, and the differentiation potency was further improved in the co-culture group containing microenvironmental cells than in the other groups **(****FIG. 13B****).**

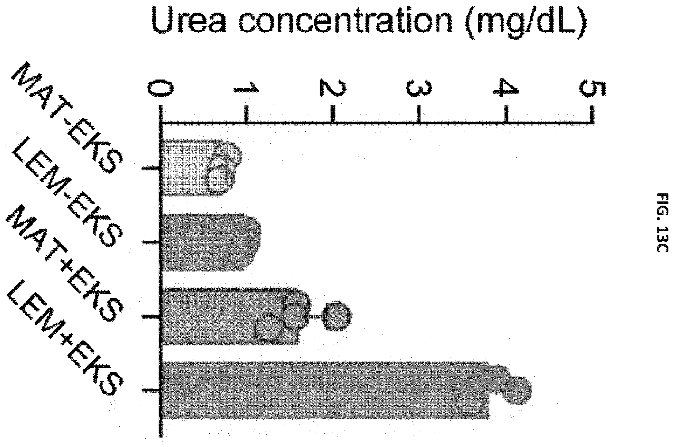

Likewise, even when liver function was compared through analysis of urea synthesis ability, which is a representative liver function indicator, it was confirmed that the urea synthesis ability of the liver organoid cultured in the LEM hydrogel was superior to that of the Matrigel group, and the urea synthesis ability was further improved in the co-culture group containing microenvironmental cells than in the other groups **(****FIG. 13C****).** Accordingly, it can be seen that the liver organoid model integrated with the liver tissue-specific microenvironment in the LEM hydrogel condition is most superior in liver differentiation and functionality.

### Test Example 3-4. Comparison of liver differentiation and functionality of human iPSC-derived liver organoid with or without co-culture of microenvironmental cell

**To** check whether a hepatic differentiation marker and functionality can be improved when Kupffer cells (K) and hepatic stellate cells (S) present in the liver in addition to iPSC-derived hepatocytes (H), vascular endothelial cells (E), and mesenchymal stem cells (M) contained in the prepared iPSC-derived liver organoids are further co-cultured, these microenvironmental cells were further contained in human iPSC-derived liver organoids (HEM) cultured in the decellularized liver tissue-derived scaffold (LEM) and cultured together (HEMKS). Specifically, in the case of co-cultured liver organoids (H:E:M:K:S), organoids were prepared by mixing a total of 400,000 cells at a ratio of 10:7:2:2:1. A group (HEM) in which these microenvironmental cells were not co-cultured was used as a control group. Cells differentiated from human induced pluripotent stem cells (iPSCs) were used as Kupffer cells and hepatic stellate cells. Immunostaining and quantitative PCR were performed 5 days after co-culture to compare the HEM group and the HEMKS group.

**As** a result of immunostaining of CD68, a Kupffer cell marker, and PDGFRB, GFAP, FN, and SMA, hepatic stellate cell markers, it was confirmed that markers of each microenvironmental cell were not expressed in the HEM organoids, but markers specific to the corresponding cells were expressed in the HEMKS organoids **(****FIG. 14A****).** In particular, as a result of comparing the expression of SOX17, AFP, and ALB to check whether the expression of hepatic differentiation markers is further improved by the Kupffer cells and hepatic stellate cells, it was confirmed that the expression of hepatic differentiation markers also increased in the HEMKS group in which the Kupffer cells and hepatic stellate cells were co-cultured together.

Further, as a result of quantitative PCR to compare the expression of each marker gene of the HEMKS organoid group in which the liver-specific microenvironment was constructed and the HEM organoid group without the microenvironmental cells, it was confirmed that the expression of hepatic differentiation markers (HNF4A, ALB) significantly increased in the HEMKS organoids (FIG. 14B). Since the hepatic stellate cells were added to the HEMKS organoids compared to the HEM organoids, the expression of PDGFRB and COL1A1, which are mainly expressed in mature hepatic stellate cells, increased in the HEMKS group. Also, it was confirmed that the expression of vascular cells present in the HEM organoids increased more than 10-fold in the HEMKS organoids in which the microenvironmental cells were further co-cultured.

According to these results, it can be seen that the liver organoid model integrated with the liver tissue-specific microenvironment has higher liver differentiation and functionality than the conventional liver organoid model. Therefore, it is expected that more accurate disease phenotypes can be induced through non-alcoholic steatohepatitis modeling in the organoid in which the liver microenvironment is integrated.

### Test Example 4: Evaluation of drug efficacy using non-alcoholic steatohepatitis (NASH) organoid model

### Test Example 4-1. Evaluation of drug efficacy using human tissue-derived non-alcoholic steatohepatitis organoid (1)

Steatohepatitis (NASH) was induced in liver organoids in which a liver tissue microenvironment [vascular endothelial cells (E) + Kupffer cells (K) + hepatic stellate cells (S)] was constructed under culture conditions in which the decellularized liver tissue-derived LEM scaffold and the multiwell microfluidic device were combined, followed by treatment with obeticholic acid (OCA) at different concentrations. OCA (Obeticholic acid) is a semi-synthetic bile acid analogue that has conventionally been used to treat cholangitis and is a drug candidate whose efficacy has recently been verified in non-alcoholic steatohepatitis clinical trials. The NASH group was treated with 800 µM fatty acid for 3 days to induce a disease, and the NASH + OCA group was treated with 800 µM fatty acid and the corresponding drug for 3 days at different concentrations.

As a result, it was confirmed that in the fatty liver organoids (NASH) compared to the organoids of the normal group (Normal), fat accumulated and organoid-specific morphology and structure was damaged. Also, it was confirmed that in the group treated with obeticholic acid, the morphology and structure of the organoid were recovered and the amount of fat accumulation was reduced as the concentration increased **(****FIG. 15A****).**

Also, when the viability of the normal liver organoids treated with the drug was analyzed to evaluate the cytotoxicity depending on the concentration of the drug, it was confirmed that the viability was 80% or more at 1 µM, 70% at 10 µM, and about 60% at 100 µM **(****FIG. 15B****).** That is, it was confirmed that liver toxicity increased as the treatment concentration of OCA increased.

Further, as a result of quantitative PCR to compare disease marker gene expression depending on the treatment concentration of the effective drug, it was confirmed that the expression of an inflammation-related marker (TNFα) and a fibrosis-related markers (SMA) increased in the fatty liver organoid group (NASH), and decreased in response to treatment with OCA. However, the expression of the inflammation marker and fibrosis-related marker further increased at a drug concentration of 100 µM due to drug toxicity **(****FIG. 15C****).**

Accordingly, the applicability of the NASH liver organoid model co-cultured with EC, HSC, and KC cells under the LEM-Chip culture conditions was verified as an in vitro non-alcoholic steatohepatitis model for evaluation of drug efficacy and toxicity.

### Test Example 4-2. Evaluation of drug efficacy using human tissue-derived non-alcoholic steatohepatitis organoid (2)

Steatohepatitis (NASH) was induced in liver organoids in which a liver tissue microenvironment [vascular endothelial cells (E) + Kupffer cells (K) + hepatic stellate cells (S)] was constructed under culture conditions in which the decellularized liver tissue-derived LEM scaffold and the multiwell microfluidic device were combined, followed by treatment with ezetimibe (Eze) at different concentrations. Eze (Ezetimibe) is a cholesterol absorption inhibitor and is a drug candidate that has conventionally been used to treat hyperglycemic cholesterol and lipid abnormalities. The NASH group was treated with 800 µM fatty acid for 3 days to induce a disease, and the NASH + Eze group was treated with 800 µM fatty acid and the corresponding drug for 3 days at different concentrations.

As a result, it was confirmed that in the fatty liver organoids (NASH) compared to the organoids of the normal group (Normal), fat accumulated and organoid-specific morphology and structure was damaged. Also, it was confirmed that in the group treated with ezetimibe, the morphology and structure of the organoid were recovered and the amount of fat accumulation was reduced as the concentration increased **(****FIG. 16A****).**

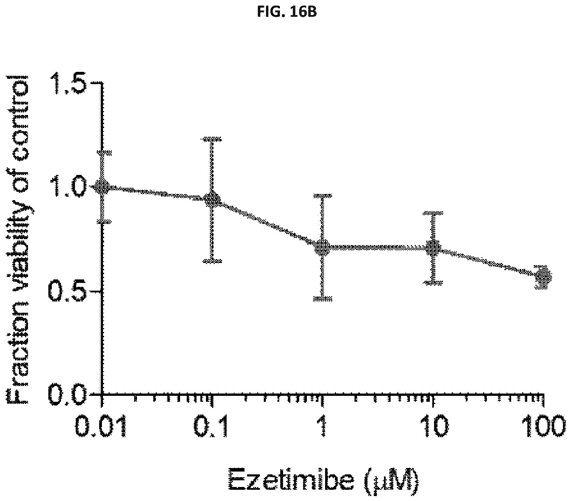

**Also,** when the viability of the normal liver organoids treated with the drug was analyzed to evaluate the cytotoxicity depending on the concentration of the drug, it was confirmed that the viability was 70% or more at 1 µM to 10 µM and about 50% at 100 µM **(****FIG. 16B****).**

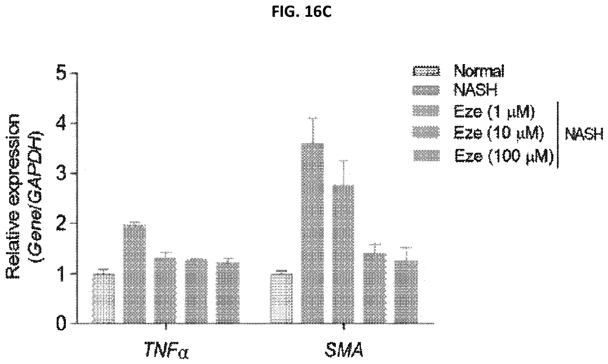

Further, as a result of quantitative PCR to compare disease marker gene expression depending on the treatment concentration of the effective drug, it was confirmed that the expression of an inflammation-related marker (TNFα) and a fibrosis-related markers (SMA) increased in the fatty liver organoid group (NASH), and decreased in response to treatment with Eze. Furthermore, in the fatty liver organoid group (NASH), the expression of the inflammation marker and fibrosis marker decreased to a level similar to that in the normal organoid at a drug concentration of 10 µM or more **(****FIG. 16C****).**

Accordingly, the applicability of the NASH liver organoid model co-cultured with EC, HSC, and KC cells under the LEM-Chip culture conditions was verified as an in vitro non-alcoholic steatohepatitis model for evaluation of drug efficacy and toxicity.

### Test Example 4-3. Evaluation of drug efficacy using human tissue-derived non-alcoholic steatohepatitis organoids (3)

Steatohepatitis (NASH) was induced in liver organoids in which a liver tissue microenvironment [vascular endothelial cells (E) + Kupffer cells (K) + hepatic stellate cells (S)] was constructed under culture conditions in which the decellularized liver tissue-derived LEM scaffold and the multiwell microfluidic device were combined, followed by treatment with dapagliflozin (DAPA) at different concentrations. DAPA (Dapagliflozin) is a sodium-glucose transporter inhibitor and is a drug candidate that has conventionally been used to treat diabetes. The NASH group was treated with 800 µM fatty acid for 3 days to induce a disease, and the NASH + DAPA group was treated with 800 µM fatty acid and the corresponding drug for 3 days at different concentrations.

**As** a result, it was confirmed that in the fatty liver organoids (NASH) compared to the organoids of the normal group (Normal), fat accumulated and organoid-specific morphology and structure was damaged. Also, it was confirmed that in the group treated with dapagliflozin, the morphology and structure of the organoid were recovered and the amount of fat accumulation was reduced as the concentration increased **(****FIG. 17A****).**

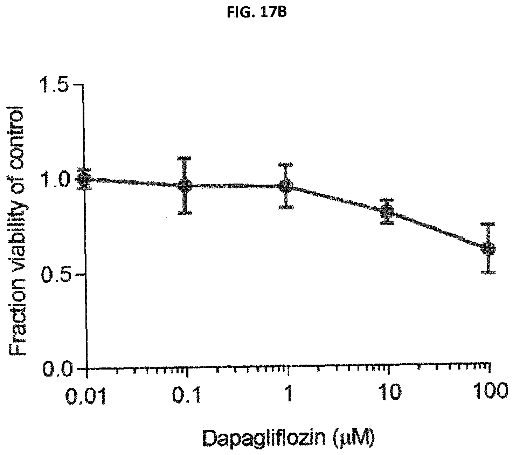

Also, when the viability of the normal liver organoids treated with the drug was analyzed to evaluate the cytotoxicity depending on the concentration of the drug, it was confirmed that the viability was not affected at a concentration of up to 1 µM, and it was 80% or more at 10 µM and about 60% at 100 µM **(****FIG. 17B****).**

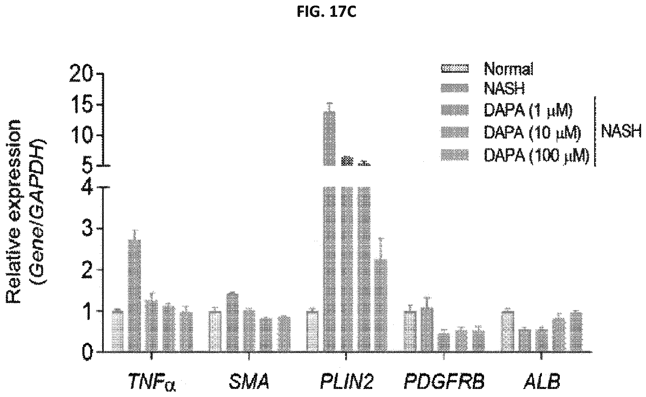

Further, as a result of quantitative PCR to compare disease marker gene expression depending on the treatment concentration of the effective drug, it was confirmed that the expression of an inflammation-related marker (TNFα), a fibrosis-related markers (SMA), a fat accumulation marker (PLIN2), and a hepatic stellate cell activation marker (PDGFRB) increased in the fatty liver organoid group (NASH), and decreased depending on the treatment concentration of DAPA. However, the expression of ALB, a hepatic differentiation marker, decreased in the fatty liver organoid group (NASH) and the functionality was recovered in response to treatment with DAPA **(****FIG. 17C****).**

Accordingly, the applicability of the NASH liver organoid model co-cultured with EC, HSC, and KC cells under the LEM-Chip culture conditions was verified as an in vitro non-alcoholic steatohepatitis model for evaluation of drug efficacy and toxicity.

### Test Example 5: Identification of mechanism and effect of selected effective drug based on non-alcoholic steatohepatitis (NASH) organoid

### Test Example 5-1. Verification of drug efficacy in human tissue-derived liver organoid-based non-alcoholic steatohepatitis model

After NASH was induced in a human tissue-derived liver organoid model integrated with the liver microenvironment in the LEM-Chip culture platform, analysis was performed to check whether steatohepatitis can be treated with the previously selected dapagliflozin (DAPA). To further verify the need for vascular cells (E), Kupffer cells (K), and hepatic stellate cells (S) to fabricate a NASH model, an organoid model in which the microenvironmental cells were not co-cultured was used as a control group.

As a result of immunostaining to compare the normal organoid group (Normal - EKS) in which the microenvironmental cells were not co-cultured and the normal group (Normal + EKS) in which the microenvironmental cells were co-cultured, it was confirmed that cells expressing CD68, a Kupffer cell marker, and α-SMA, a hepatic stellate cell marker, were distributed around the organoids in the organoid group in which the microenvironmental cells were co-cultured **(****FIG. 18A****).** When NASH was induced in the organoid group in which the microenvironmental cells were co-cultured, accumulation of some fatty acid was observed inside the organoid and in the surrounding microenvironmental cells, and a decrease in fatty acid accumulation was observed in the group treated with DAPA. Also, it was confirmed that α-SMA, a hepatic stellate cell marker and liver fibrosis marker, was expressed inside the organoid and in the surrounding matrix and immune cells in the NASH + EKS group, α-SMA was expressed in organoids, surrounding matrix, and immune cells, but the expression of α-SMA decreased in the DAPA-treated group.

**Also,** NASH was induced in each of the group in which the microenvironment was constructed and the group in which only organoids were cultured and quantitative PCR was performed to compare the expression of inflammation (IL-6, IL-1β, TNF-α), fibrosis (SMA, COL1A1, TGF-β) and de novo lipogenesis (SREBPC1, FAS, ACC)-related genes depending on the presence or absence of co-cultured cells (EKS). As a result, it was confirmed that when NASH was induced in the organoids without containing the co-cultured cells, the expression of the inflammation, fibrosis and de novo lipogenesis-related genes increased, whereas when NASH was induced in the organoids containing the co-cultured cells, the expression of each of the inflammation, fibrosis and de novo lipogenesis-related genes increased significantly **(****FIG. 18B****).** Further, compared to the normal organoids without the co-cultured cells (Normal - EKS), the normal organoids with the co-cultured cells (Normal + EKS) contain matrix cells and immune cells, which causes a difference in initial expression of the inflammation, fibrosis and de novo lipogenesis genes. Therefore, if NASH is induced in an organoid without co-cultured cells, inaccurate information regarding inflammation, fibrosis, and de novo lipogenesis may be provided. Furthermore, when the NASH organoid model integrated with the microenvironment was treated with DAPA, it was confirmed that the expression of the inflammation, fibrosis and de novo lipogenesis-related genes decreased and steatohepatitis was improved.

Accordingly, it was verified that the human tissue-derived NASH organoid model integrated with the LEM-Chip-based liver microenvironment developed in the present disclosure can present a more accurate non-alcoholic steatohepatitis disease phenotype than conventional organoid models, and the newly discovered DAPA based on the model showed an effective therapeutic effect on steatohepatitis.

### Test Example 5-2. Verification of drug efficacy in human iPSC-derived liver organoid-based non-alcoholic steatohepatitis model (1)

To verify the drug efficacy of dapagliflozin (DAPA) in the human iPSC-derived liver organoid (HEMKS) steatohepatitis model cultured in the LEM-Chip platform, the Normal group, the group treated with 800 µM oleic acid for 5 days to induce NASH, and the NASH + DAPA group treated with 800 µM oleic acid and 50 µM DAPA were compared and analyzed. The NASH organoid model was subjected to immunostaining and western blot analysis for markers related to inflammation, fibrosis, and mTORC1 mechanism.

**As** a result of immunostaining to compare the expression of SMA, a fibrosis-related marker, ACC, a de novo lipogenesis-related marker, and p-S6, and p-4EBP1, mTORC1 mechanism-related markers, it was confirmed that these markers significantly increased in the NASH group compared to the normal organoids and decreased in the group treated with DAPA **(****FIG. 19A****).** Staining of CD68, a Kupffer cell marker, confirmed that Kupffer cells were mainly distributed in an area where a lot of inflammation occurred and p-S6 was overexpressed in the NASH group. As a result of BODIPY staining for labelling ALB, a liver function marker, and accumulated fatty acid, the accumulated fatty acid increased and ALB decreased in the NASH group, whereas the accumulated fatty acid decreased and ALB increased in the group treated with DAPA, which confirmed an improvement in liver function.

Also, as a result of western blot analysis to identify the expression of each marker protein, it was confirmed that the expression of fibrosis-related markers (SMA, Col1A1), inflammation-related markers (IL1-β, p-lkB/lkB), and mTORC1-related markers (p-4EBP1/4EBP1, p-p70S6K1/p70S6K1, p-S6/S6) increased in the NASH organoids and decreased in the group treated with DAPA **(****FIG. 19B****).**

**Accordingly,** it was verified that inflammation and fibrosis increased in the human iPSC-derived NASH organoid model fabricated under the LEM-Chip culture conditions similar to those in the actual steatohepatitis, whereas the expression of inflammation, fibrosis and mTORC1-related proteins was recovered to an almost normal level by the newly discovered DAPA.

### Test Example 5-3. Verification of drug efficacy in human iPSC-derived liver organoid-based non-alcoholic steatohepatitis model (2)

**To** verify the drug efficacy of dapagliflozin (DAPA) in the human iPSC-derived liver organoid (HEMKS) steatohepatitis model cultured in the LEM-Chip platform, the Normal group, the group treated with 800 µM oleic acid for 5 days to induce NASH, and the NASH + DAPA group treated with 800 µM oleic acid and 50 µM DAPA were compared and analyzed.

As a result of Masson's Trichrome (MT) staining to check collagen accumulated inside the organoid due to fibrosis, the amount of collagen accumulation caused by fibrosis was significantly increased in the NASH group compared to the normal organoid group and was decreased in the group treated with DAPA, which was confirmed through histological staining and quantitative analysis (FIG. 20A).

Also, as a result of Oil red O staining, which stains fatty acids accumulated in cells, it was confirmed that the accumulation of fat stained red inside the organoids significantly increased and the accumulation of fatty acid decreased in the group treated with DAPA **(****FIG. 20B****).**

Accordingly, it was confirmed that the efficacy of DAPA can be verified in the human iPSC-derived liver organoid (HEMKS) steatohepatitis model cultured in the LEM-Chip platform.

### Test Example 5-4. Comparison in efficacy of effective drug and identification of therapeutic mechanism using human tissue-derived acute and chronic non-alcoholic steatohepatitis organoid models (1)

To identify the therapeutical mechanism of dapagliflozin (DAPA) in the human tissue-derived liver organoid steatohepatitis (NASH) model cultured in the LEM-Chip platform and verify the drug efficacy, the Normal group, the group in which NASH was induced, and the NASH + DAPA group were compared and analyzed. An acute NASH model was divided into a group in which organoids were treated with 800 µM oleic acid for 3 days and a group in which organoids were treated with 800 µM oleic acid + 50 µM DAPA for 3 days, and a chronic NASH model was divided into a group in which organoids were treated with 800 µM oleic acid+ 20 ng/ml TGF-β for 5 days and a group in which organoids were treated with 800 µM oleic acid + 20 ng/ml TGF-β + 50 µM DAPA for 5 days. The human tissue-derived acute and chronic NASH organoid models were subjected to western blot analysis for markers related to inflammation, fibrosis, YAP/TAZ, and mTORC1 mechanism.

**As** a result of western blot analysis to compare the expression of fibrosis markers α-SMA and COL1A1, inflammation markers p-lkB/lkB and pro-IL1β, a YAP/TAZ mechanism-related marker p-YAP/YAP and mTORC1 mechanism-related markers p-S6/S6, p-p70S6K1/p70S6K1 and p-4EBP1/4EBP1, the expression of protein markers related to fibrosis and inflammation significantly increased in both the acute and chronic NASH groups compared to the normal organoids, and decreased in the group treated with DAPA **(****FIG. 21A****).** However, it was confirmed that the expression of the YAP/TAZ mechanism-related protein and the mTORC1 mechanism-related proteins increased in the acute NASH organoids and decreased in the DAPA-treated group, but remarkably decreased in the chronic NASH organoids and recovered to an almost normal level by DAPA.

Further, even when quantification was performed through three replicate tests, it was confirmed that the expression of markers related to fibrosis and inflammation was significantly decreased by DAPA in both the acute and chronic NASH models **(****FIG. 21B****).** However, the expression of YAP/TAZ mechanism-related protein and the mTORC1 mechanism-related proteins increased in the acute model and significantly decreased in the chronic model, but recovered to an almost normal level by DAPA.

**Accordingly,** it was confirmed that when acute and chronic non-alcoholic steatohepatitis were induced in human tissue-derived organoid models, the expression of YAP/TAZ and mTORC1-related proteins increased in the acute model and decreased in the chronic model, but the overexpression or underexpression of the proteins was recovered to an almost normal level by DAPA, which is an effective drug newly discovered in the present disclosure. Therefore, it is verified that the human NASH organoid model constructed in the present disclosure can be used not only for discovering an effective drug for NASH treatment, but also for research to identify the therapeutic mechanism at the molecular level of the drug.

### Test Example 5-5. Comparison in efficacy of effective drug and identification of therapeutic mechanism using human tissue-derived acute and chronic non-alcoholic steatohepatitis organoid models (2)

To confirm the reproducibility of the protein expression results previously confirmed by western blot analysis for the human tissue-derived acute and chronic NASH organoid models cultured in the LEM-Chip platform, immunostaining was performed for markers related to liver function, fibrosis, and mTORC1 mechanism.

To efficiently induce acute and chronic NASH in the human tissue-derived organoid model cultured in the LEM-Chip platform, an optimized model was constructed by varying treatment concentrations of fatty acid, treatment periods, and fibrosis-inducing cytokine treatment. The acute NASH model was prepared by treating an organoid with only 800 µM oleic acid for 3 days as in the previous condition, and the chronic NASH model was prepared by treating an organoid with 800 µM oleic acid and 20 ng/ml TGF-β, a fibrotic cytokine, for 5 days. In the DAPA drug test, the acute model was treated with 800 µM Oleic acid + 50 µM DAPA for 3 days and the chronic model was treated with 800 µM Oleic acid + 20 ng/ml TGF-β + 50 µM DAPA for 5 days **(****FIG. 22A****).**

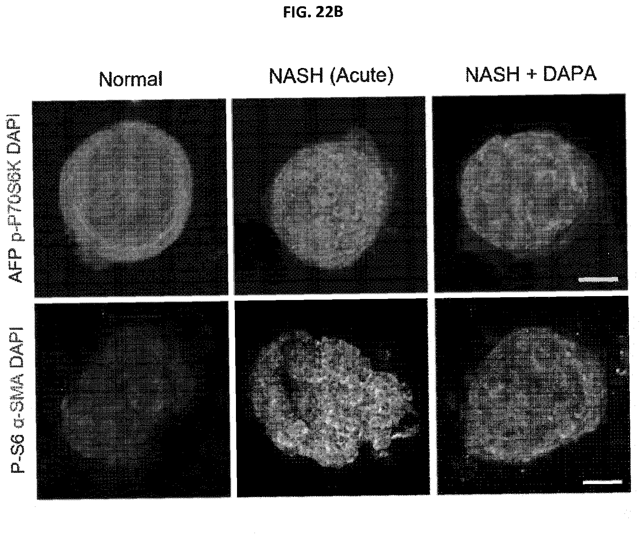

As a result of immunostaining on the acute model induced by treating the human tissue-derived NASH organoid model with the fatty acid for 3 days, it was confirmed that the expression of AFP, a liver function-related marker, decreased in the NASH organoid model, but partially recovered in the DAPA-treated group, and the expression of α-SMA, a fibrosis-related marker, increased in the NASH-induced group and decreased in the DAPA-treated group. Also, it was confirmed that the expression of p-S6 and p-P70S6K1 related to the mTORC1 mechanism increased in the acute NASH model compared to the normal organoids and decreased due to DAPA **(****FIG. 22B****).**

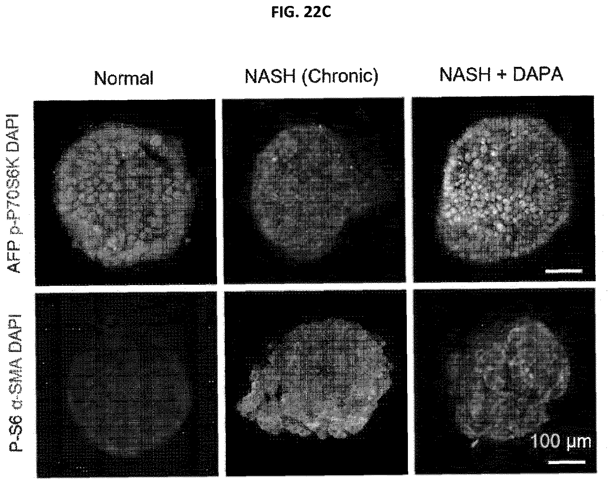

As a result of immunostaining on the chronic model induced by treating the human tissue-derived NASH organoid model with the fatty acid and TGF-β for 5 days, it was confirmed that the expression of AFP, a liver function-related marker, decreased in the NASH organoid model, but partially recovered in the DAPA-treated group, and the expression of α-SMA, a fibrosis-related marker, increased in the NASH-induced group and decreased in the DAPA-treated group. In particular, it can be seen that the expression of α-SMA greatly increased in the chronic NASH-induced model rather than in the acute NASH-induced model. Also, it was confirmed that the expression of p-S6 and p-P70S6K1 related to the mTORC1 mechanism decreased in the chronic NASH model compared to the normal organoids and was recovered by DAPA **(****FIG. 22C****).**

Accordingly, it was confirmed that when acute and chronic non-alcoholic steatohepatitis were induced in human tissue-derived organoid models, the expression of mTORC1-related proteins increased in the acute model and decreased in the chronic model, which is consistent with the result of western blot analysis. Also, it was confirmed that the overexpression or underexpression of the proteins was recovered to an almost normal level by DAPA, which is a newly discovered effective drug.

Test Example 6: Efficacy evaluation of novel therapeutic drug discovered in non-alcoholic steatohepatitis (NASH) organoid model and verification of related mechanism (Evaluation of drug efficacy and mechanism research in NASH animal model)

### Test Example 6-1. Verification of DAPA efficacy in non-alcoholic steatohepatitis animal model (1)

To check whether dapagliflozin (DAPA) which was discovered through a steatohepatitis (NASH) organoid model and whose therapeutic efficacy was verified is also effective in an actual NASH animal model, early non-alcoholic steatohepatitis was induced in mice and DAPA was administered once at a concentration of 5 mg/kg on day 2.

A Fa/R (fasting overnight, refed with high-carbohydrate, fat-free diet) model induced in the present test can well simulate de novo lipogenesis of an actual patient with non-alcoholic steatohepatitis and was prepared by not feeding for one day and then acutely inducing steatohepatitis with a high-carbohydrate/fat-free diet for another day. DAPA was orally administered once at a concentration of 5 mg/kg at the time of administration of the Fa/R feed **(****FIG. 23A****).**

When liver tissues were collected from the mice of the normal, Fa/R, and Fa/R + DAPA groups and subjected to H&E staining and MT staining, it was confirmed that a lot of fatty acid accumulation occurred in the liver tissues of the Fa/R group and collagen was accumulated therein due to fibrosis. In contrast, the accumulation of fatty acid decreased and fibrosis was improved in the DAPA-administered group. Also, as a result of quantitative analysis to measure the collagen-stained area, it was confirmed that the collagen area decreased in the liver tissue of the DAPA-administered group (**FIG. 23B****).**

Further, 3-nitrotyrosine (3-NT) was accumulated due to an increase in reactive oxygen species in the liver and highly expressed in the liver tissue with steatohepatitis or fibrosis. To confirm the effect of reducing oxidative stress by DAPA, protein components of the liver tissue were extracted and subjected to western whole blotting analysis for 3-NT. As a result, it was confirmed that the expression of 3-NT increased in the Fa/R group, but significantly decreased in the DAPA-treated group **(****FIG. 23C****).**

The liver is responsible for detoxifying lipopolysaccharide (LPS), and when fatty liver is induced, the function of hepatocytes decreases and the amount of serum LPS increases. Since the amount of collagen accumulation increases as fibrosis occurs, hydroxyproline, which is present in a large amount in the collagen increased due to fibrosis, is also used as an indicator of fibrosis caused by steatohepatitis. Serum LPS and albumin were measured by hematology analysis through whole blood collection on day 3, and the concentration of hydroxyproline was quantified through ELISA analysis. It was confirmed that the amounts of LPS and hydroxyproline increased in the Fa/R group and decreased in the DAPA-treated group compared to the normal group **(****FIG. 23D****).** Also, it can be seen that serum albumin, as an indicator of liver function, decreased in the Fa/R group and was recovered by DAPA.

Accordingly, it was verified that DAPA whose efficacy was verified in the NASH organoid model was also effective in an animal model in which acute early non-alcoholic steatohepatitis was induced, which confirmed that the NASH organoid model constructed in the present disclosure can be applied as an in vitro model for discovering therapeutic substances for steatohepatitis.

### Test Example 6-2. Verification of DAPA efficacy in non-alcoholic steatohepatitis animal model (2)

To check whether dapagliflozin (DAPA) whose efficacy was verified in a steatohepatitis (NASH) organoid model is also effective in an actual NASH animal model, early non-alcoholic steatohepatitis was induced in mice and the drug was orally administered (5 mg/kg). Analysis was performed on Day 3 when model induction and drug administration were completed.

**As** a result of TUNEL staining on the liver tissues of the mice of the normal, Fa/R, and Fa/R + DAPA groups to detect apoptosis, the intensity of TUNEL increased in the Fa/R group and decreased due to DAPA, which confirmed that necrosis of tissues caused by steatohepatitis was inhibited by DAPA **(****FIG. 24A****).**

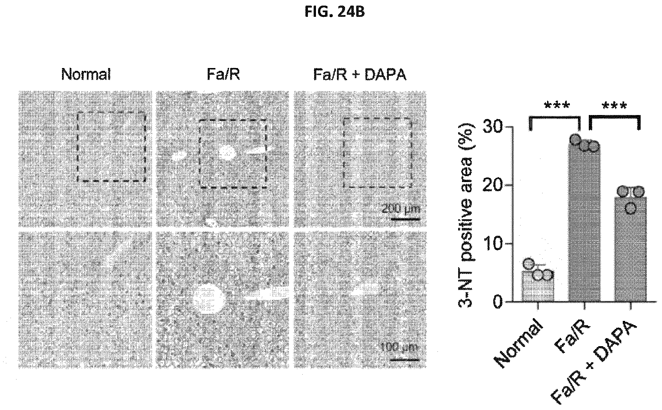

**To** confirm the effect of reducing oxidative stress by DAPA, 3-nitrotyrosine (3-NT) in the liver tissue was subjected to immunohistochemistry analysis. As a result, it was confirmed that the number of cells expressing 3-NT increased in the Fa/R group due to ballooning of hepatocytes, but significantly decreased in the DAPA-treated group **(****FIG. 24B****).**

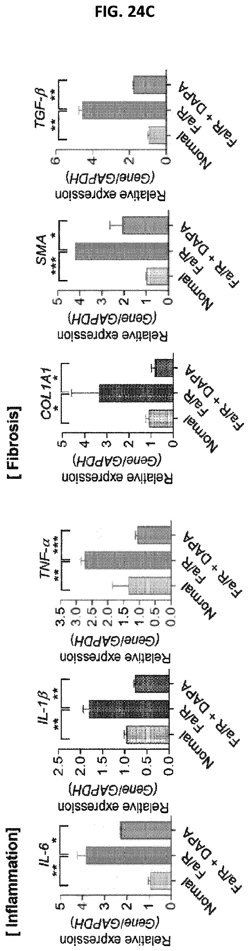

**As** a result of quantitative PCR on the liver tissue of each group, it was confirmed that the expression of inflammation-related IL-6, IL-1β, and TNF-α and the expression of fibrosis-related COL1A1, SMA, and TGF-β increased in the Fa/R group, which is an acute NASH model, and decreased due to DAPA **(****FIG. 24C****).**

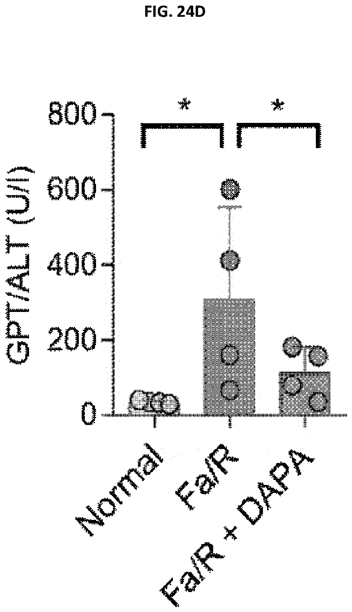

As a result of analyzing ALT, an indicator of liver toxicity, through hematology analysis, it was confirmed that the ALT level increased about 10-fold in the Fa/R model compared to the normal mouse group, but significantly decreased due to DAPA **(****FIG. 24D****).**

Accordingly, it was verified that DAPA whose efficacy was verified in the NASH organoid model was effective in an animal model in which acute non-alcoholic steatohepatitis was induced.

### Example 6-3. Verification of DAPA efficacy in non-alcoholic steatohepatitis animal model (3)

To check whether dapagliflozin (DAPA) whose efficacy was verified in a steatohepatitis (NASH) organoid model is also effective in an actual NASH animal model, non-alcoholic steatohepatitis with severe lesions was induced in mice and the drug was administered.

A CDAHFD (choline-deficient, L-amino acid-defined, high-fat diet) model induced in the present test can well simulate NASH-related fibrosis of a patient with severe non-alcoholic steatohepatitis and was prepared by chronically inducing steatohepatitis with a high-fat, choline-deficient diet for eight weeks. DAPA was orally administered at a concentration of 5 mg/kg three times a week from week 4 after the CDAHFD diet was induced (FIG. 25A).

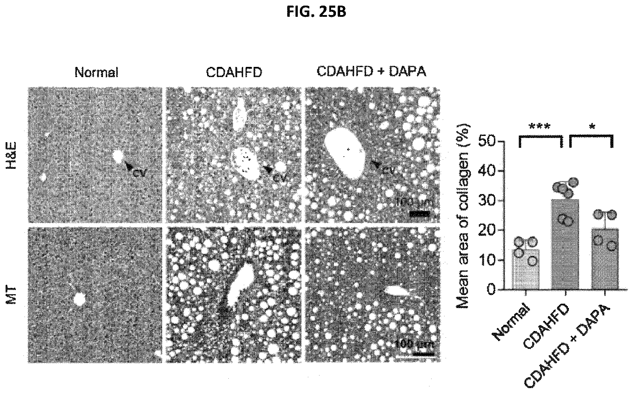

When liver tissues were collected from the mice of the normal, CDAHFD, and CDAHFD + DAPA groups at week 8 and subjected to H&E staining and MT staining, it was confirmed that severe accumulation of fatty acid occurred in the CDAHFD group and collagen was excessively accumulated therein due to fibrosis. Accordingly, it can be seen that fibrosis is more strongly induced in the CDAHFD model than in the above-described Fa/R model. In contrast, the accumulation of fat decreased and fibrosis was improved in the DAPA-administered group. Also, as a result of quantitative analysis to measure the collagen-stained area, it was confirmed that the area decreased in the DAPA-administered group (cv: central vein) (FIG. 25B).

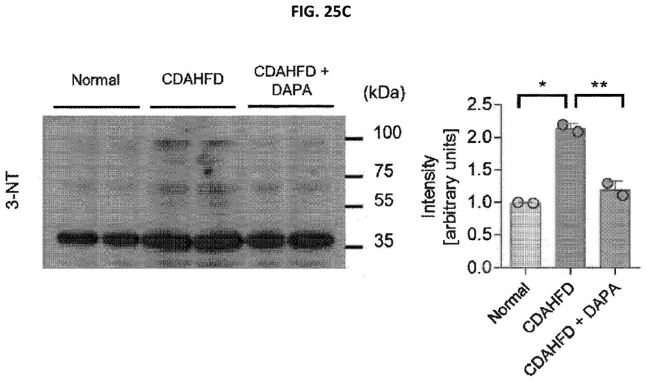

Further, 3-nitrotyrosine (3-NT) was accumulated due to an increase in reactive oxygen species in the liver and highly expressed in the liver tissue with steatohepatitis or fibrosis. To confirm the effect of reducing oxidative stress by DAPA, protein components of the liver tissue were extracted and subjected to western whole blotting analysis for 3-NT. As a result, it was confirmed that the expression of 3-NT increased in the CDAHFD group, but significantly decreased in the DAPA-treated group **(****FIG. 25C****).**

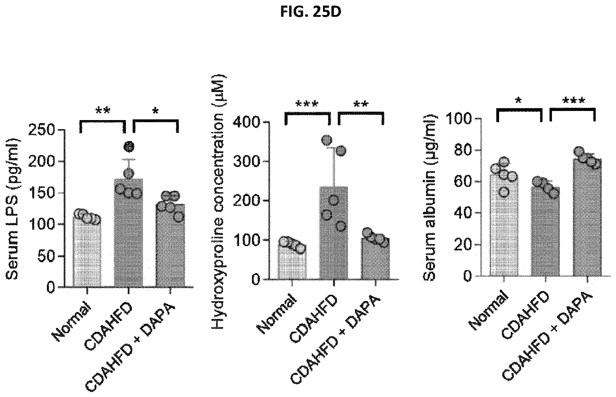

When the amounts of serum LPS and hydroxyproline, which are indicators of liver toxicity, were measured at week 8 in the same manner as in the above-described Fa/R model, it was confirmed that the amounts of serum LPS and hydroxyproline increased in the CDAHFD group and decreased in the DAPA-treated group compared to the normal group. Also, it can be seen that serum albumin, as an indicator of liver function, decreased in the CDAHFD group and was recovered by DAPA **(****FIG. 25D****).**

**Accordingly,** it was verified that DAPA whose efficacy was verified in the NASH organoid model was also effective in an animal model in which chronic non-alcoholic steatohepatitis was induced, which confirmed that the NASH organoid model constructed in the present disclosure can be applied as an in vitro model for discovering therapeutic substances for steatohepatitis.

### Test Example 6-4. Verification of DAPA efficacy in non-alcoholic steatohepatitis animal model (4)

To check whether dapagliflozin (DAPA) whose efficacy was verified in a steatohepatitis (NASH) organoid model is also effective in an actual NASH animal model, non-alcoholic steatohepatitis with severe lesions was induced in mice and the drug was administered. The drug was orally administered at a concentration of 5 mg/kg three times a week from week 4. Quantitative PCR was performed at week 8 at the end of the test.

When the size of the liver tissue of each group was measured at week 8 at the end of the test, the CDAHFD chronic non-alcoholic steatohepatitis model showed severe ballooning of the liver tissue and increased in volume, but the liver tissue of the DAPA-treated group showed a decrease in ballooning **(****FIG. 26A****).**

When liver tissues were collected from the mice of the normal, CDAHFD, and CDAHFD + DAPA groups at week 8 and subjected to quantitative PCR, it was confirmed that the expression of inflammation-related IL-6, IL-1β, and TNF-α and the expression of fibrosis-related COL1A1, SMA, and TGF-β increased in the CDAHFD group, which is a chronic NASH model, and decreased due to DAPA **(****FIG. 26B****).**

Accordingly, it was verified that DAPA whose efficacy was verified in the NASH organoid model was also effective in improving steatohepatitis in all of animal models in which acute early NASH or chronic NASH was induced.

### Test Example 6-5. Comparison in efficacy of effective drug and identification of therapeutic mechanism in acute and chronic non-alcoholic steatohepatitis animal models (1)

To verify whether DAPA whose efficacy was verified in acute and chronic non-alcoholic steatohepatitis animal models is also effective in animal models according to the same therapeutic mechanism as identified in the above-described acute and chronic NASH liver organoid models, western blot analysis was performed, followed by marker quantification based on the western blot analysis. The acute and chronic non-alcoholic steatohepatitis animal models were prepared by inducing NASH with the Fa/R and CDAHFD feed, respectively.

As a result of western blot analysis to compare the expression of fibrosis-related α-SMA and COL1A1, inflammation-related p-lkB/lkB and pro-IL1β, mTORC1 mechanism-related p-S6/S6, p-p70S6K1/p70S6K1, and p-4EBP1/4EBP1, it was confirmed that the expression of fibrosis and inflammation-related marker proteins significantly increased in the liver tissues of both the acute (Fa/R) and chronic (CDAHFD) NASH groups compared to the liver tissues of the normal mice, and the expression of the markers decreased in the DAPA-treated group. Also, the expression of mTORC1 mechanism-related proteins increased in the acute NASH animal model (Fa/R) and decreased in the DAPA-treated group, whereas the expression of mTORC1-related protein expression significantly decreased in the chronic NASH animal model (CDAHFD) and was recovered to an almost normal level by DAPA **(****FIG. 27A****).**

When quantitative analysis was performed through repeated western blot analysis, the amounts of fibrosis-related proteins and inflammation-related proteins increased in the acute and chronic models and were lowered by DAPA, and the expression levels of mTORC1-related proteins, which increased in the acute model, were recovered by DAPA. In contrast, the expression levels of mTORC1-related proteins significantly decreased in the chronic model and was recovered by DAPA. However, in the chronic NASH animal model unlike the chronic NASH organoid model, the expression level of p-4EBP1/4EBP1 was not significantly decreased by DAPA **(****FIG. 27B****).**

Accordingly, it was confirmed that DAPA, which is a newly discovered effective drug, showed approximately the same therapeutic mechanism related to mTORC1 in the acute and chronic animal models as in the human tissue-derived acute and chronic NASH organoid models, and the overexpression or underexpression of the proteins was recovered to an almost normal level by DAPA.

### Test Example 6-6. Comparison in efficacy of effective drug and identification of therapeutic mechanism in acute and chronic non-alcoholic steatohepatitis animal models (2)

DAPA-treated liver tissues in the acute and chronic non-alcoholic steatohepatitis animal models were subjected to western blot analysis as described above, followed by immunostaining to check whether protein expression patterns of the respective markers are matched. The acute and chronic non-alcoholic steatohepatitis animal models were prepared by inducing NASH with the Fa/R and CDAHFD feed, respectively.

When fibrosis-related α-SMA was stained through immunostaining, it was confirmed that the expression of α-SMA increased in the Fa/R and CDAHFD models compared to the normal liver tissue and decreased due to DAPA. Through staining of p-S6, a mTORC1 mechanism-related protein, it was confirmed that the expression of the marker increased in the acute model as verified above and decreased due to DAPA, and the expression decreased in the chronic model was recovered by DAPA. Through immunostaining of p-4EBP1, another mTORC1 mechanism-related protein, it was confirmed that the expression of p-4EBP1 increased in both the acute and chronic NASH models and decreased due to DAPA, which is consistent with the above-described result of western blot analysis (FIG. 28).

Accordingly, the result of immunostaining confirmed that DAPA, which is a newly discovered effective drug, showed approximately the same therapeutic mechanism related to mTORC1 in the acute and chronic animal models as in the human tissue-derived acute and chronic NASH organoid models.

The present disclosure has been described with reference to the preferred exemplary embodiments thereof. It can be understood by a person with ordinary skill in the art that the present disclosure can be implemented as being modified and changed within the scope departing from the spirit and the scope of the present disclosure. Accordingly, the above-described exemplary embodiments should be considered in descriptive sense only and not for purposes of limitation. Also, the technical scope of the present disclosure is defined not by the detailed description of the invention but by the appended claims, and all differences within the scope will be construed as being comprised in the present disclosure.

## Claims

1. A non-alcoholic fatty liver artificial tissue model, comprising:
a hydrogel containing a decellularized liver tissue-derived extracellular matrix (Liver Extracellular Matrix; LEM);
a liver organoid;
a device including a well in which the hydrogel is located and a plurality of microchannels through which a free fatty acid flows; and
a culture medium containing a free fatty acid.

2. The non-alcoholic fatty liver artificial tissue model of Claim 1,
wherein the LEM is a matrix in which 95% to 99.9% of liver tissue cells have been removed.

3. The non-alcoholic fatty liver artificial tissue model of Claim 1,
wherein the liver organoid is derived from a human induced pluripotent stem cell (hiPSC) or human liver tissue.

4. The non-alcoholic fatty liver artificial tissue model of Claim 3,
wherein when the liver organoid is derived from the human induced pluripotent stem cell (hiPSC), the liver organoid is cultured on the hydrogel.

5. The non-alcoholic fatty liver artificial tissue model of Claim 3,
wherein when the liver organoid is derived from the human liver tissue, the liver organoid is cultured within the hydrogel.

6. The non-alcoholic fatty liver artificial tissue model of Claim 4,
wherein the well in which the hydrogel is located has a depth of 2 mm to 4 mm.

7. The non-alcoholic fatty liver artificial tissue model of Claim 5,
wherein the well in which the hydrogel is located has a depth of 0.5 mm to 1.5 mm.

8. The non-alcoholic fatty liver artificial tissue model of Claim 1,
wherein the free fatty acid has a concentration of 100 µM to 900 µM.

9. The non-alcoholic fatty liver artificial tissue model of Claim 1,
wherein the liver organoid is co-cultured with any one or more of vascular cells, mesenchymal stem cells, Kupffer cells (KC), and hepatic stellate cells (HSC).

10. A method of fabricating a non-alcoholic fatty liver artificial tissue model, comprising:
a process of fabricating the non-alcoholic fatty liver artificial tissue model of Claim 1; and
a process of perfusing the non-alcoholic fatty liver artificial tissue model with a culture medium containing a free fatty acid.

11. The method of fabricating a non-alcoholic fatty liver artificial tissue model of Claim 10,
wherein the process of perfusing is performed by shaking the non-alcoholic fatty liver artificial tissue model on a rocking shaker.

12. A method of screening a therapeutic drug for a non-alcoholic fatty liver disease, comprising:
a process of treating the non-alcoholic fatty liver artificial tissue model of Claim 1 with a candidate substance; and
a process of comparing a group treated with the candidate substance and a control group.
